# EUROPEAN PATENT APPLICATION

(11) **EP 4 800 033 A1**
(43) Date of publication of application: **02.09.2026**
(21) Application number: 24881953.4
(22) Date of filing: 07.06.2024
(51) Int. Cl.: C07K 16/28, A61K 39/395, A61K 47/68, A61P 35/00, A61P 43/00, C12N 1/15, C12N 1/19, C12N 1/21, C12N 5/10, C12N 15/13, C12N 15/63

(54) **HUMANIZED ANTI-CKAP4 ANTIBODY OR ANTIGEN-BINDING FRAGMENT THEREOF**

(30) Priority: 25.10.2023 JP 2023182946
(71) Applicant: The University of Osaka, Osaka 565-0871 (JP)
(72) Inventor: KIKUCHI, Akira, Suita-shi, Osaka 565-0871 (JP); SADA, Ryota, Suita-shi, Osaka 565-0871 (JP)
(74) Representative: Marks & Clerk LLP
(86) International application number: PCT/JP2024/020940
(87) International publication number: WO 2025/088835

(57) **Abstract**

The present invention addresses the problem of providing a humanized anti-CKAP4 antibody, or an antigen-biding fragment thereof, that shows strong binding to CKAP4 and that has antitumor activity. The present invention solves this problem by a humanized anti-CKAP4 antibody, or an antigen-binding fragment thereof, comprising a heavy chain variable region that comprises a heavy chain CDR1 comprising the amino acid sequence represented by SEQ ID NO: 1 or 6, a heavy chain CDR2 comprising the amino acid sequence represented by SEQ ID NO: 2 or 7, and a heavy chain CDR3 comprising the amino acid sequence represented by SEQ ID NO: 3 or 8, and that comprises an amino acid sequence having at least 90% identity to the amino acid sequence represented by SEQ ID NO: 4 or 9, and comprising a light chain variable region that comprises a light chain CDR1 comprising the amino acid sequence represented by SEQ ID NO: 11, a light chain CDR2 comprising the amino acid sequence represented by SEQ ID NO: 12, and a light chain CDR3 comprising the amino acid sequence represented by SEQ ID NO: 13, and that comprises an amino acid sequence having at least 90% identity to the amino acid sequence represented by SEQ ID NO: 14.

## Description

### Technical Field

The present invention relates to a humanized anti-CKAP4 antibody or an antigen-binding fragment thereof.

### Background Art

Dickkopf1 (DKK1) is an extracellular secreted protein that inhibits Wnt signaling. DKK1 binds to the Wnt ligand receptors LRP5/6 and inhibits Wnt signaling either by competitively blocking ligand binding or by sequestering LRP5/6 away from the cell membrane. On the other hand, while DKK1 has been known to promote the proliferation of various cancers, its molecular mechanism remained unclear for many years. Under such circumstances, the present inventors identified Cytoskeleton-associated protein 4 (CKAP4) as a receptor for DKK1. CKAP4 was originally reported as a type II transmembrane protein that is mainly present in the endoplasmic reticulum and is involved in maintaining the structure of the endoplasmic reticulum. DKK1 binds to CKAP4 localized on the plasma membrane, thereby activating the phosphatidylinositol 3-kinase (PI3K)-AKT pathway and promoting cancer cell proliferation. The co-expression of DKK1 and CKAP4 has been associated with poor prognosis in pancreatic caner, lung caner, esophageal caner, and liver cancer.

The phenotype of CKAP4 knockout (KO) mice is normal and CKAP4 KO mice are indistinguishable from their heterozygous and wild-type littermates from birth to 18 months of age. Detailed histological analysis of various organs in CKAP4 KO mice revealed no differences from wild-type mice. Therefore, therapies targeting CKAP4 were expected not to cause severe adverse effects, and a mouse anti-CKAP4 monoclonal antibody was produced (Patent Literature (PTL) 1). The mouse anti-CKAP4 antibody showed the activity of inhibiting the binding of DKK1 to CKAP4, and the activity of suppressing AKT activation, cell proliferation, and migration in pancreatic, lung, esophageal, and liver cancer cell lines in vitro. Furthermore, the mouse anti-CKAP4 antibody suppressed xenograft tumor formation by these cancer cells in nude mice (T-cell-deficient mice) and prolonged the survival of mice injected intraperitoneally with pancreatic cancer cells.

Further, the inventors found that CKAP4, a transmembrane protein, is secreted from pancreatic cancer cells and lung cancer cells together with extracellular vesicles (also known as exosomes). The incorporation of CKAP4 into exosomes was mediated by a DKK1-dependent endocytosis pathway and required exosome biosynthesis molecules. The inventors developed an ELISA that can detect CKAP4 secreted from tumors using the mouse anti-CKAP4 antibodies. Analysis of patient samples using the same CKAP4 ELISA revealed that serum CKAP4 levels were elevated in pancreatic cancer and lung cancer patients compared to healthy individuals. It was also shown that serum CKAP4 levels in patients whose cancer tissues were immunohistochemically determined to be CKAP4-positive were higher than those in patients with immunohistochemically CKAP4-negative. Further, when the serum CKAP4 levels before and after surgery in the same patients were compared, the postoperative serum CKAP4 values were much lower than the preoperative serum CKAP4 values. Since anti-CKAP4 antibodies are theoretically expected to suppress the proliferation of cancer cells that express CKAP4 on the cell membrane and secrete DKK1, detecting CKAP4 and DKK1 in serum is expected to be useful for predicting patients who will respond to treatment with anti-CKAP4 antibodies.

### Citation List

### Patent Literature

PTL 1: WO2019/065747

### Summary of Invention

### Technical Problem

Anti-CKAP4 antibodies are expected to serve as research and diagnostic tools; however, none have been approved as antibody therapeutic agents for cancer. In order to successfully develop a mouse anti-CKAP4 antibody as a therapeutic agent, it is necessary to humanize the antibody, which can reduce the possibility of patients producing neutralizing antibodies against non-human molecules. However, when humanized, the antibodies often lose their binding affinity or their intended *in vivo* activity.

An object of the present invention is to provide a humanized anti-CKAP4 antibody or antigen-binding fragment thereof that shows high binding affinity to CKAP4 and that has antitumor activity.

### Solution to Problem

As a result of diligent research in view of the above problem, the present inventors found that the above problem can be solved by a humanized anti-CKAP4 antibody or an antigen-binding fragment thereof, comprising:
a heavy chain variable region that comprises
   heavy chain CDR1 comprising the amino acid sequence represented by SEQ ID NO: 1 or 6,
   heavy chain CDR2 comprising the amino acid sequence represented by SEQ ID NO: 2 or 7, and
   heavy chain CDR3 comprising the amino acid sequence represented by SEQ ID NO: 3 or 8, and that comprises
   an amino acid sequence having at least 90% identity to the amino acid sequence represented by SEQ ID NO: 4 or 9; and
a light chain variable region that comprises
   light chain CDR1 comprising the amino acid sequence represented by SEQ ID NO: 11,
   light chain CDR2 comprising the amino acid sequence represented by SEQ ID NO: 12, and
   light chain CDR3 comprising the amino acid sequence represented by SEQ ID NO: 13, and that comprises
   an amino acid sequence having at least 90% identity to the amino acid sequence represented by SEQ ID NO: 14.
As a result of further research based on this finding, the present inventors have accomplished the present invention. Specifically, the present invention includes the following aspects.

Item 1. A humanized anti-CKAP4 antibody, or an antigen-binding fragment thereof, comprising:
a heavy chain variable region that comprises
   a heavy chain CDR1 comprising the amino acid sequence represented by SEQ ID NO: 1 or 6,
   a heavy chain CDR2 comprising the amino acid sequence represented by SEQ ID NO: 2 or 7, and
   a heavy chain CDR3 comprising the amino acid sequence represented by SEQ ID NO: 3 or 8, and that comprises
   an amino acid sequence having at least 90% identity to the amino acid sequence represented by SEQ ID NO: 4 or 9; and
a light chain variable region that comprises
   a light chain CDR1 comprising the amino acid sequence represented by SEQ ID NO: 11,
   a light chain CDR2 comprising the amino acid sequence represented by SEQ ID NO: 12, and
   a light chain CDR3 comprising the amino acid sequence represented by SEQ ID NO: 13, and that comprises
   an amino acid sequence having at least 90% identity to the amino acid sequence represented by SEQ ID NO: 14.

Item 2. The humanized anti-CKAP4 antibody or antigen-binding fragment thereof according to Item 1, wherein at least one of the identities is at least 95%.

Item 3. The humanized anti-CKAP4 antibody or antigen-binding fragment thereof according to Item 1 or 2, wherein at least one of the identities is 100%.

Item 4. The humanized anti-CKAP4 antibody or antigen-binding fragment thereof according to any one of Items 1 to 3,
wherein
the heavy chain variable region comprises the amino acid sequence represented by SEQ ID NO: 4 or 9, and
the light chain variable region comprises the amino acid sequence represented by SEQ ID NO: 14.

Item 5. The humanized anti-CKAP4 antibody or antigen-binding fragment thereof according to any one of Items 1 to 4, comprising
a heavy chain constant region that comprises an amino acid sequence having at least 90% identity to the amino acid sequence represented by SEQ ID NO: 16, and
a light chain constant region that comprises an amino acid sequence having at least 90% identity to the amino acid sequence represented by SEQ ID NO: 17.

Item 6. A polynucleotide comprising a coding sequence for the humanized anti-CKAP4 antibody or antigen-binding fragment thereof of any one of Items 1 to 5.

Item 7. The polynucleotide according to Item 6, which is an expression vector.

Item 8. A cell comprising the polynucleotide of Item 6 or 7.

Item 9. A pharmaceutical composition comprising the humanized anti-CKAP4 antibody or antigen-binding fragment thereof of any one of Items 1 to 5.

Item 10. A reagent comprising the humanized anti-CKAP4 antibody or antigen-binding fragment thereof of any one of Items 1 to 5.

Item 11. An antitumor agent comprising the humanized anti-CKAP4 antibody or antigen-binding fragment thereof of any one of Items 1 to 5.

Item 11A. A method for treating cancer, comprising administering the humanized anti-CKAP4 antibody or antigen-binding fragment thereof of any one of Items 1 to 5 to a subject having cancer.

Item 11B. The humanized anti-CKAP4 antibody or antigen-binding fragment thereof according to any one of Items 1 to 5, for use in the treatment of cancer.

Item 11C. Use of the humanized anti-CKAP4 antibody or antigen-binding fragment thereof of any one of Items 1 to 5 in producing an antitumor agent.

Item 11D. Use of the humanized anti-CKAP4 antibody or antigen-binding fragment thereof of any one of Items 1 to 5 as an antitumor agent.

Item 12. The antitumor agent according to Item 11, wherein the humanized anti-CKAP4 antibody or antigen-binding fragment thereof is a conjugate with a drug, or is for use in co-administration with a drug.

Item 13. An antitumor immune response activator comprising the humanized anti-CKAP4 antibody or antigen-binding fragment thereof of any one of Items 1 to 5.

Item 13A. A method for activating an antitumor immune response, comprising administering the humanized anti-CKAP4 antibody or antigen-binding fragment thereof of any one of Items 1 to 5 to a subject having cancer.

Item 13B. The humanized anti-CKAP4 antibody or antigen-binding fragment thereof according to any one of Items 1 to 5, for use in activating an antitumor immune response.

Item 13C. Use of the humanized anti-CKAP4 antibody or antigen-binding fragment thereof according to any one of Items 1 to 5 in producing an antitumor immune response activator.

Item 13D. Use of the humanized anti-CKAP4 antibody or antigen-binding fragment thereof of any one of Items 1 to 5 as an antitumor immune response activator.

### Advantageous Effects of Invention

According to the present invention, a humanized anti-CKAP4 antibody or an antigen-binding fragment thereof that shows strong binding to CKAP4 and that has antitumor activity can be provided.

### Brief Description of Drawings

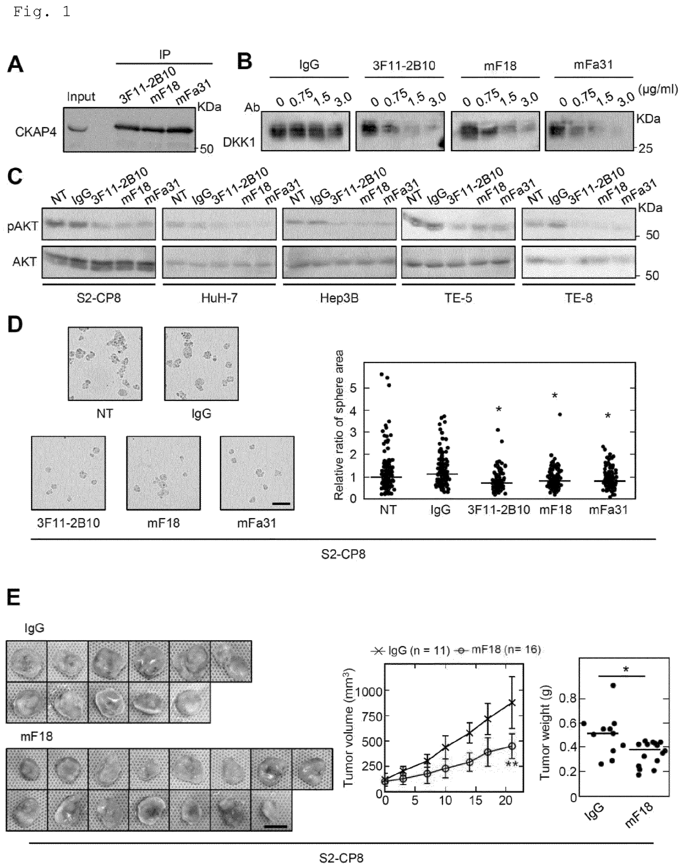

Fig. 1 shows the results of characterization of silenced Fc mouse anti-CKAP antibodies (mF18, mFa31) with mutations introduced into the Fc region, which mean the antibodies that do not bind to complement. (A) The lysate (input) of S2-CP8 cells was immunoprecipitated with 1 µg/mL of anti-CKAP4 antibody (3F11-2B10, mF18, mFa31). The input and immunoprecipitates (IP) were detected with a rabbit anti-CKAP4 polyclonal antibody. (B) After 2 nM GST-CKAP4-ECD (extracellular domain) was pretreated with the indicated anti-CKAP4 antibodies or control IgG at indicated concentrations, the mixture was mixed with 2.5 nM DKK1 for 1 hour. The protein mixture was precipitated with glutathione agarose, and the precipitate was detected with an anti-DKK1 antibody. (C) Lysates of various cancer cells treated with 20 µg/mL of indicated anti-CKAP4 antibodies or control IgG for 4 hours were detected with the labeled antibody. (D) Left panel: S2-CP8 cells treated with 20 µg/mL of labeled anti-CKAP4 antibody or control IgG were cultured in 3D Matrigel for 5 days.
   Right panel: The total area of tumor spheres in three fields of view was measured and presented as a dot plot. The size of tumor spheres was expressed as a ratio relative to that under untreated conditions (with the median being set to 1)*. *P < 0.05 (Student's t-test)*. (E) S2-CP8 cells were subcutaneously transplanted into both dorsal flanks of immunodeficient mice. The mice were intraperitoneally injected with either silent Fc mouse anti-CKAP4 antibody (200 µg/dose, N = 15) or control IgG (N = 11) twice a week. Excised xenograft tumors (left panel), tumor volumes (center panel), and tumor weights (right panel) are shown. **P < 0.05*; ***P < 0.01 (Student's t-test).* Scale bars: 100µm (D), 10mm (E)*.*

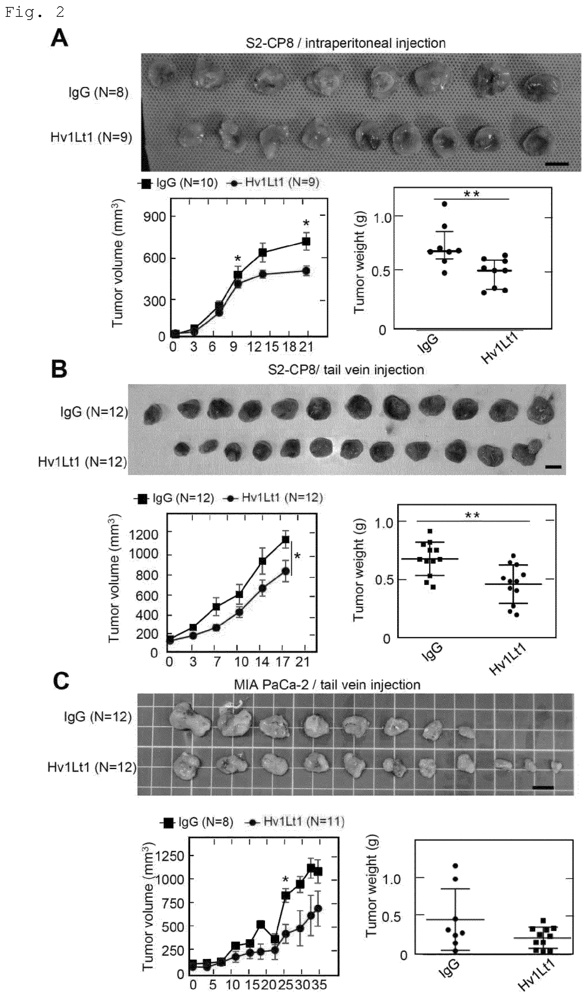

Fig. 2 shows the results of an in vivo tumor growth inhibition assay using the humanized anti-CKAP4 antibody Hv1Lt1. S2-CP8 cells (A and B) or MIA PaCa-2 cells (C) were subcutaneously transplanted into both dorsal flanks of immunodeficient mice. The mice were intraperitoneally injected with either humanized anti-CKAP4 antibody Hv1Lt1 or control IgG (A, 200 µg/dose) or intravenously injected via the tail vein (B and C, 400 µg/dose) twice a week. Excised xenograft tumors (upper panel), tumor volumes (lower-left panel), and weights (lower right panel) are shown. In the figure, N indicates the number of tumors under each assay condition*. *P < 0.05; **P < 0.01 (Student's t-test)*, scale bar: 10 mm.

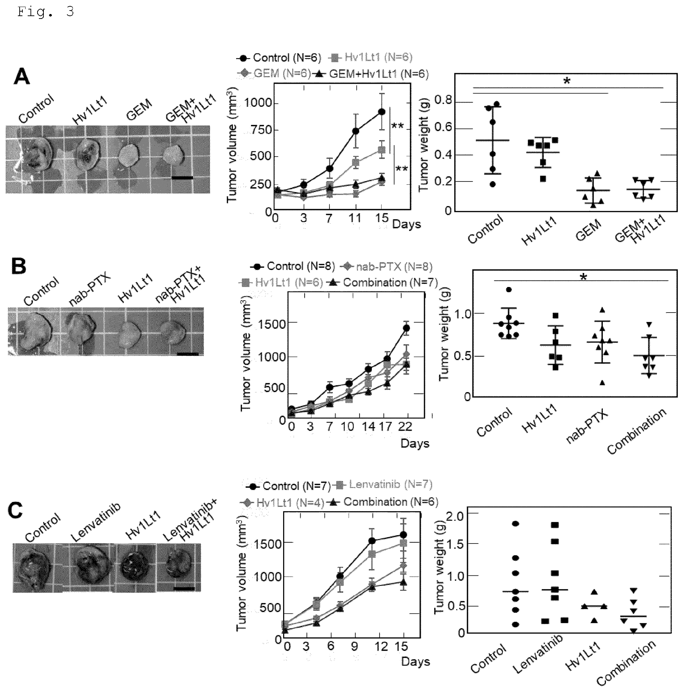

Fig. 3 shows the test results of in vivo tumor growth inhibitory effects of the combination of humanized anti-CKAP4 antibody Hv1Lt1 with a standard chemotherapeutic agent.
   (A) S2-CP8 cells were subcutaneously inoculated into both dorsal flanks of immunodeficient mice and randomly divided into four groups: a control group (control IgG + phosphate-buffered saline (PBS)), an Hv1Lt1 group (Hv1Lt1 + PBS), a GEM group (control IgG + gemcitabine), and a GEM + Hv1Lt1 group (Hv1Lt1 + gemcitabine). While 280 µg of antibody was intraperitoneally administered to each mouse twice a week and 100 µg of gemcitabine (or PBS) was intraperitoneally administered once a week, tumor growth was observed for 15 days.
   (B) S2-CP8 cells were subcutaneously inoculated into both dorsal flanks of immunodeficient mice, and the mice were randomly divided into four groups: a control group (control IgG + physiological saline), an Hv1Lt1 group (Hv1Lt1 + physiological saline), a nab-PTX group (control IgG + nab-PTX), and a nab-PTX + Hv1Lt1 group (Hv1Lt1 + nab-PTX). 280 µg of the antibody and 10 mg/kg of the nab-PTX (or saline) were intraperitoneally administered to each mouse twice a week, and tumor growth was observed for 22 days.
   (C) Hep3B cells were subcutaneously inoculated into both dorsal flanks of immunodeficient mice, and the mice were randomly divided into four groups:
      a control group (control IgG + a 0.5% (wt/vol) methylcellulose 400 solution),
      an Hv1Lt1 group (Hv1Lt1 + a 0.5% (wt/vol) methylcellulose 400 solution),
      a lenvatinib group (control IgG + lenvatinib), and
      an Hv1Lt1 + lenvatinib group (Hv1Lt1 + lenvatinib).
      While 1 mg/kg of lenvatinib or the 0.5% (wt/vol) methyl cellulose 400 solution was orally administered to each mouse once a day, 6 days a week, and 280 µg of antibody was intraperitoneally administered twice a week, tumor growth was observed for 15 days. The excised xenograft tumors (left panel), tumor volume (left central panel), and body weight (right panel) are shown. In the figure, N indicates the number of tumors under each assay condition*. *P < 0.05; **P < 0.01 (ANOVA post-hoc test)*; scale bar: 10 mm.

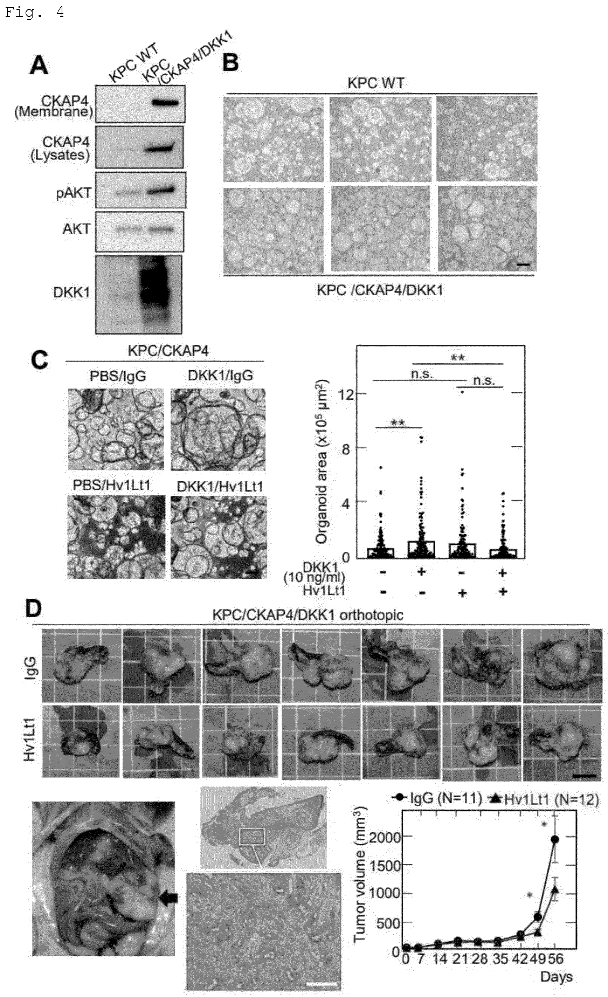

Fig. 4 shows the in vitro and in vivo growth inhibitory effects of Hv1Lt1 on pancreatic cancer organoids.
   (A) Wild-type (WT) KPC organoids (organoids prepared from pancreatic cancer that developed in LSL-KrasG12D/+; Trp53fl/fl; Elastase-Cre mice) and KPC organoids stably expressing CKAP4 and DKK1 (KPC/CKAP4/DKK1) were biotinylated, and cell surface proteins were precipitated using NeutrAvidin agarose beads. The precipitate (Membrane) and cell lysate were detected with the indicated antibodies.
   (B) WT KPC organoids and KPC/CKAP4/DKK1 organoids were dissociated into single cells, mounted in Matrigel, and cultured in complete medium. Organoids at day 4 are shown (three fields of view for each organoid).
   (C) Left panel: KPC/CKAP4 organoids were cultured with 10 ng/mL of purified mouse DKK1-FLAG in 3D Matrigel for 5 days. At the time of embedding the organoids in Matrigel, 10 µg/mL of Hv1Lt1 or control IgG was added to the culture medium. Right panel: The total area/field of organoid spheres (N = three fields or view, top 50 spheres per well) was measured, and the area of each sphere is shown as a dot plot. Each bar graph shows the average value*. **P < 0.01 (ANOVA post-hoc test)*.
   (D) KPC/CKAP4/DKK1 organoids were orthotopically transplanted into the pancreatic tail of immunodeficient mice. Hv1Lt1 or control IgG was injected intraperitoneally twice a week (280 µg/body). The progression of tumor development was observed for 56 days. Excised tumors (upper panel), macroscopic appearance of KPC tumors (lower-left panel, red arrow), hematoxylin- and eosin-stained resected tumor sections (lower center), and tumor volumes over time (lower-right panel) are shown. N indicates the number of tumors determined by assay. Results are presented as mean ± SEM. **P < 0.05 (Student's t-test);* scale bars: 500 µm (B and C) and 10 mm*.*

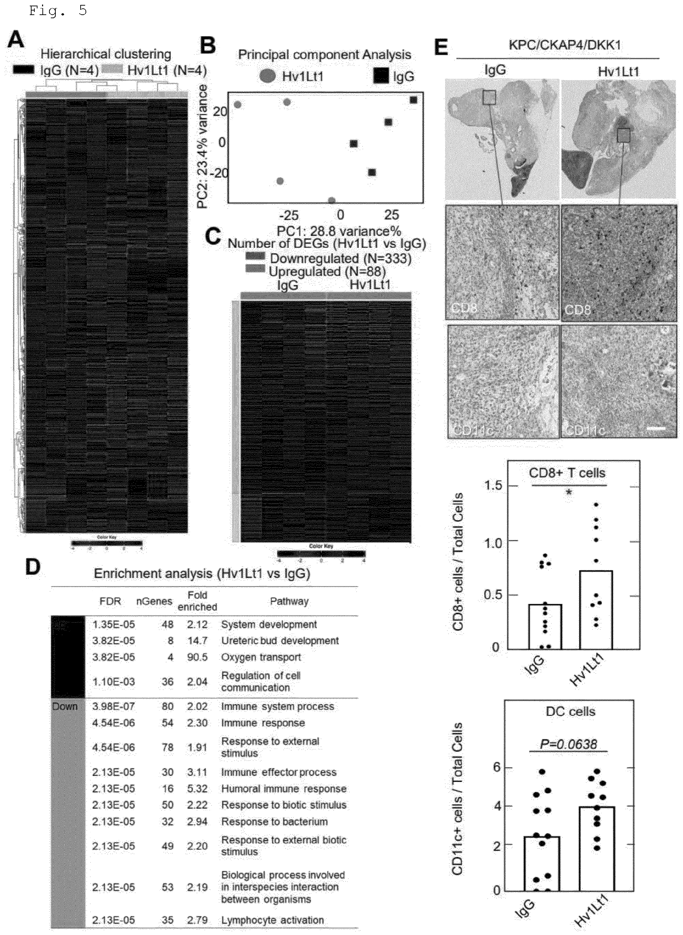

Fig. 5 shows the results of transcriptome analysis of pancreatic tumors treated with Hv1Lt1.
   (A) The heatmap shows the z-score of each row (gene) of the gene expression data (four control tumors and four Hv1Lt1-treated tumors used in Fig. 4D). The results of the hierarchical clustering are shown in a dendrogram.
   (B) A PCA (principal component analysis) plot of RNA-sequence data is shown.
   (C) The heatmap shows the z-scores for each row (gene) of gene expression data of 421 differentially expressed genes (DEGs). In Hv1Lt1-treated tumors, 88 DEG genes increased, while 333 DEG genes decreased.
   (D) The results of the GO molecular function enrichment analysis for the DEGs in Fig. 5C are shown.
      FDR: false discovery rate, nGenes: number of DEGs included in the indicated pathway.
   (E) Immunohistochemical staining was performed on tissue sections from 12 control tumors and 10 Hv1Lt1-treated tumors, and the numbers of immune cells infiltrating the tumors were evaluated. Upper panels**:** HE staining images and anti-CD8 antibody staining images (staining CD8-positive T cells) and anti-CD11c antibody staining images (staining dendritic cells) of one representative control tumor and one representative Hv1Lt1-treated tumor are shown. Lower panels: The numbers of CD8-positive and CD11c-positive cells relative to the total number of intratumoral cells were quantified and presented as dot plots. Statistical comparisons were performed between the control and Hv1Lt1-treated tumors. **P < 0.05 (Student's t-test).* Scale bar: 250 µm (E).

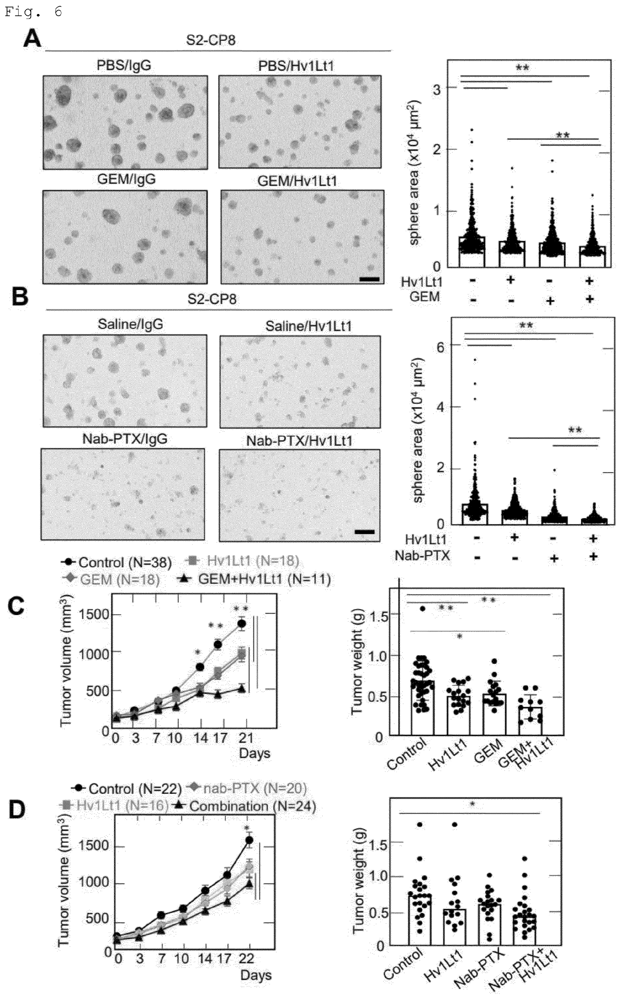

Fig. 6 shows the tumor growth-inhibitory effect of the combination of Hv1Lt1 and a standard chemotherapeutic agent.
   (A) Left panel: S2-CP8 cells were treated with a medium containing 20 µg/mL of the indicated anti-CKAP4 antibody or control IgG, together with 1nM gemcitabine (GEM) or an equivalent volume of PBS, and then cultured in 3D Matrigel for 6 days. Right panel: The areas of the top 150 largest tumor spheres from three fields of view were measured and presented as dot plots. ***P < 0.01 (ANOVA post-hoc test).*
   (B) Left panel: S2-CP8 cells were treated with a medium containing 20 µg/mL of the indicated anti-CKAP4 antibody or control IgG, together with 10 nM nanoparticle albumin-bound paclitaxel (nab-PTX) or an equivalent volume of physiological saline, and then cultured in 3D Matrigel for 7 days. Right panel: The areas of the top 150 largest tumor spheres from three fields of view were measured and presented as dot plots. **P < 0.01 (ANOVA post-hoc test).*
   (C, D) After subcutaneous inoculation of S2-CP8 cells into both dorsal flanks of mice, the mice were randomly divided into four groups. While (C) 2 mg/kg of gemcitabine was administered once a week or (D) 10 mg/kg of nab-PTX was administered twice a week, 280 µg of Hv1Lt1 was administered twice a week. The progression of tumor development was monitored for 21 days. Tumor volumes (left panel) and body weights (right panel) are shown. N indicates the number of tumors counted in each assay. The results are presented as mean ± SEM;
   * *P < 0.05; ** P < 0.01 (ANOVA post-hoc test)*, Scale bar: 10 mm.

### Description of Embodiments

In the present specification, the expressions "containing" and "comprising" include the concepts of "containing," "comprising," "consisting essentially of," and "consisting only of."

### 1. Antibody or Antigen-Binding Fragment Thereof

In one embodiment, the present invention relates to a humanized anti-CKAP4 antibody or an antigen-binding fragment thereof (which may be referred to herein as "the antibody of the present invention"). This is explained below.

The antibody of the present invention has the property of binding to CKAP4.

CKAP4 is an expression product of the CKAP4 gene. For example, CKAP4 is in the form of a protein expressed in organisms. The biological species from which CKAP4 is derived is not particularly limited, and examples include animals, such as various mammals including humans, monkeys, mice, rats, dogs, cats, rabbits, pigs, horses, cattle, sheep, goats, and deer. Among these, humans are particularly preferred.

The amino acid sequences of CKAP4 derived from various biological species are either known or can be easily identified by identity analysis based on known amino acid sequences. Specifically, for example, human CKAP4 includes a protein consisting of the amino acid sequence represented by SEQ ID NO: 25.

CKAP4 may have amino acid mutations such as substitutions, deletions, additions, and insertions that may exist between individuals. Preferable examples of mutations include substitutions, and more preferably conservative substitutions.

In the present specification, "conservative substitution" means that an amino acid residue is substituted with an amino acid residue having a similar side chain. For example, substitution among amino acid residues having basic side chains such as lysine, arginine, and histidine corresponds to conservative substitution. Further, substitutions among amino acid residues having acidic side chains such as aspartic acid and glutamic acid; amino acid residues having uncharged polar side chains such as glycine, asparagine, glutamine, serine, threonine, tyrosine, and cysteine; amino acid residues having nonpolar side chains such as alanine, valine, leucine, isoleucine, proline, phenylalanine, methionine, and tryptophan; amino acid residues having β-branched side chains such as threonine, valine, and isoleucine; and amino acid residues having aromatic side chains such as tyrosine, phenylalanine, tryptophan, and histidine also correspond to conservative substitutions.

Preferred specific examples of CKAP4 include at least one selected from the group consisting of proteins defined below in (a) and proteins defined below in (b):
(a) proteins comprising the amino acid sequence represented by SEQ ID NO: 25, and
(b) proteins comprising an amino acid sequence having at least 85% identity to the amino acid sequence represented by SEQ ID NO: 25, and having the ability to interact with the extracellular secreted protein DKK1.

The presence or absence of the ability to interact with DKK1 can be determined based on criteria well known to those skilled in the art by an in vitro test method capable of evaluating the bond between a receptor and a ligand.

In the present specification, "identity" of amino acid sequences refers to the degree of matching between two or more comparable amino acid sequences. Therefore, the higher the matching between two amino acid sequences, the higher the identity or similarity of those sequences. The level of identity of amino acids sequences is determined, for example, using FASTA, a sequence analysis tool, with default parameters. Alternatively, the level of identity can also be determined by using the BLAST algorithm developed by Karlin and Altschul (Karlin S., Altschul S. F. "Methods for assessing the statistical significance of molecular sequence features by using general scoring schemes" Proc Natl Acad Sci USA.87: 2264-2268 (1990); Karlin S., Altschul S. F. "Applications and statistics for multiple high-scoring segments in molecular sequences" Proc Natl Acad Sci USA. 90: 5873-5877 (1993)). A program called BLASTX based on such a BLAST algorithm has been developed. Specific procedures for these analytical methods are known and can be referred to on the website of the National Center of Biotechnology Information (NCBI) (http://www.ncbi.nlm.nih.gov/). The "identity" of base sequences is also defined in the same manner as above.

In the above (b), the identity is more preferably 90% or higher, even more preferably at least 95%, and still even more preferably at least 98%.

Examples of the protein described above in (b) include (b') a protein comprising an amino acid sequence in which one or multiple amino acids are substituted, deleted, added, or inserted, relative to the amino acid sequence represented by SEQ ID NO: 25, and having the ability to interact with the extracellular secreted protein DKK1.

In the above (b'), the term "multiple" means, for example, a number of 2 to 20, preferably 2 to 10, more preferably 2 to 5, and even more preferably 2 or 3.

The antibody of the present invention comprises:
a heavy chain variable region that comprises
   a heavy chain CDR1 comprising the amino acid sequence represented by SEQ ID NO: 1 or 6,
   a heavy chain CDR2 comprising the amino acid sequence represented by SEQ ID NO: 2 or 7, and
   a heavy chain CDR3 comprising the amino acid sequence represented by SEQ ID NO: 3 or 8, and that comprises
   an amino acid sequence having at least 90% (preferably at least 95%, more preferably at least 98%, still more preferably at least 99%, and particularly preferably 100%) identity to the amino acid sequence represented by SEQ ID NO: 4 or 9; and
a light chain variable region that comprises
   a light chain CDR1 comprising the amino acid sequence represented by SEQ ID NO: 11,
   a light chain CDR2 comprising the amino acid sequence represented by SEQ ID NO: 12, and
   a light chain CDR3 comprising the amino acid sequence represented by SEQ ID NO: 13, and that comprises
   an amino acid sequence having at least 90% (preferably at least 95%, more preferably at least 98%, still more preferably at least 99%, and particularly preferably 100%) identity to the amino acid sequence represented by SEQ ID NO: 14.

In a particularly preferred embodiment of the present invention, the present invention relates to a humanized anti-CKAP4 antibody, or an antigen-binding fragment thereof, comprising a heavy chain variable region comprising the amino acid sequence represented by SEQ ID NO: 4 or 9, and a light chain variable region comprising the amino acid sequence represented by SEQ ID NO: 14.

In one embodiment, the antibody of the present invention comprises either (a) or (b):
(a)
   a heavy chain variable region that comprises
      a heavy chain CDR1 comprising the amino acid sequence represented by SEQ ID NO: 1,
      a heavy chain CDR2 comprising the amino acid sequence represented by SEQ ID NO: 2, and
      a heavy chain CDR3 comprising the amino acid sequence represented by SEQ ID NO: 3, and that comprises
      an amino acid sequence that comprises and has 90% or higher (preferably at least 95%, more preferably at least 98%, still more preferably at least 99%, particularly preferably 100%) identity to the amino acid sequence represented by SEQ ID NO: 4, and
   a light chain variable region comprising
      a light chain CDR1 comprising the amino acid sequence represented by SEQ ID NO: 11,
      a light chain CDR2 comprising the amino acid sequence represented by SEQ ID NO: 12, and
      a light chain CDR3 comprising the amino acid sequence represented by SEQ ID NO: 13, and and that comprises
      an amino acid sequence having at least 90% (preferably at least 95%, more preferably at least 98%, still more preferably at least 99%, and particularly preferably 100%) identity to the amino acid sequence represented by SEQ ID NO: 14;
      or
(b)
   a heavy chain variable region that comprises
      a heavy chain CDR1 comprising the amino acid sequence represented by SEQ ID NO: 6,
      a heavy chain CDR2 comprising the amino acid sequence represented by SEQ ID NO: 7, and
      a heavy chain CDR3 comprising the amino acid sequence represented by SEQ ID NO: 8, and
         that comprises
      an amino acid sequence having at least 90% (preferably at least 95%, more preferably at least 98%, still more preferably at least 99%, and particularly preferably 100%) identity to the amino acid sequence represented by SEQ ID NO: 9, and
   a light chain variable region comprising
      a light chain CDR1 comprising the amino acid sequence represented by SEQ ID NO: 11,
      a light chain CDR2 comprising the amino acid sequence represented by SEQ ID NO: 12, and
      a light chain CDR3 comprising the amino acid sequence represented by SEQ ID NO: 13; and
         that comprises
      an amino acid sequence having at least 90% (preferably at least 95%, more preferably at least 98%, still more preferably at least 99%, and particularly preferably 100%) identity to the amino acid sequence represented by SEQ ID NO: 14.

From the standpoint of its ability to inhibit sphere formation, the antibody of the present invention is particularly preferably antibody (a).

Since the antibody of the present invention is a humanized anti-CKAP4 antibody, the constant region is a sequence derived from a human antibody.

In a preferable embodiment of the present invention, the antibody of the present invention comprises a heavy chain signal peptide comprising an amino acid sequence having at least 90% (preferably at least 95%, more preferably at least 98%, still more preferably at least 99%, and particularly preferably 100%) identity to the amino acid sequence represented by SEQ ID NO: 30, as a heavy chain signal peptide.

In a preferable embodiment of the present invention, the antibody of the present invention comprises a light chain signal peptide comprising an amino acid sequence having at least 90% (preferably at least 95%, more preferably at least 98%, still more preferably at least 99%, and particularly preferably 100%) identity to the amino acid sequence represented by SEQ ID NO: 31.

It is particularly preferable that the signal peptide is located at the N-terminus of the heavy chain/light chain.

In a preferable embodiment of the present invention, the antibody of the present invention has a heavy chain constant region derived from human IgG1 as the heavy chain constant region and has a light chain constant region derived from human IgK as the light chain constant region. In a more preferable embodiment of the present invention, the antibody of the present invention comprises a heavy chain constant region comprising an amino acid sequence having at least 90% (preferably at least 95%, more preferably at least 98%, still more preferably at least 99%, and particularly preferably 100%) identity to the amino acid sequence represented by SEQ ID NO: 16, and a light chain constant region comprising an amino acid sequence having at least 90% (preferably at least 95%, more preferably at least 98%, even more preferably at least 99%, and particularly preferably 100%) identity to the amino acid sequence represented by SEQ ID NO: 17.

The constant region is typically located adjacent to the C-terminus of the variable region.

The antibody of the present invention is intended to encompass both monoclonal and polyclonal antibodies. The antibody of the present invention can be of any isotype, such as IgG (e.g., IgG1, IgG2, IgG3, and IgG4), IgA (e.g., IgA1 and IgA2), IgD, IgE, or IgM. The antibody of the present invention is preferably a monoclonal antibody.

In the present specification, the antigen-binding fragment of an antibody is not particularly limited as long as it contains a heavy chain variable region and a light chain variable region. Examples include Fab, F(ab')₂, minibody, scFv-Fc, Fv, scFv, diabody, triabody, and tetrabody.

A Fab has a structure comprising a heavy-chain fragment comprising a heavy-chain variable region and CH1 in a heavy-chain constant region and a light chain comprising a light chain variable region and a light chain constant region (CL), wherein the heavy chain variable region and the light chain variable region associate through the non-covalent intermolecular interactions described above or are linked via a disulfide bond. In a Fab, the CH1 and CL may also be connected by a disulfide bond formed between the thiol groups of the cysteine residues present in each domain.

A F(ab')₂ has a structure in which two pairs of the Fab fragments described above are present, and the CH1 domains are linked to each other via a disulfide bond formed between the thiol groups of the cysteine residues contained therein.

A minibody has a structure comprising two fragments in which a CH3 domain is fused to the heavy-chain variable region that constitutes the scFv described below, and the two CH3 domains associate with each other through non-covalent intermolecular interactions.

scFv-Fc has a structure in which two antibody fragments, each containing scFv, CH2, and CH3 shown below, associate through non-covalent binding intermolecular interactions between their CH3 domains, as in the minibody described above, an a disulfide bond is formed between the thiol groups of the cysteine residues within each CH3 domain.

An Fv is also referred to as the smallest structural unit of an antibody, and is a structure in which a heavy chain variable region and a light chain variable region are associated through non-covalent intermolecular interactions. In an Fv, the thiol groups of cysteine residues present in the heavy chain variable region and the light chain variable region may form a disulfide bond.

An scFv is a structure in which the C-terminus of the heavy-chain variable region is linked to the N-terminus of the light-chain variable region by a linker, or conversely, the N-terminus of the heavy-chain variable region is linked to the C-terminus of the light-chain variable region by a linker. It is also referred to as a single-chain antibody.

A diabody, triabody, and tetrabody are structures in which two, three, and four scFv units form a dimer, a trimer, and a tetramer, respectively. Similar to Fv fragments, these oligomers associate into structurally stable assemblies through non-covalent intermolecular interactions between their variable regions.

The antibody of the present invention may also be conjugated or fused to other peptides, oligopeptides, or proteins. Examples of such other peptides, oligopeptides, or proteins include albumin (e.g., serum albumin), protein tags (e.g., biotin, His tag, FLAG tag, Halo tag, MBP tag, HA tag, Myc tag, V5 tag, PA tag), fluorescent proteins (e.g., GFP, Azami-Green, ZsGreen, GFP2, HyPer, Sirius, BFP, CFP, Turquoise, Cyan, TFP1, YFP, Venus, ZsYellow, Banana, KusabiraOrange, RFP, DsRed, AsRed, Strawberry, Jred, KillerRed, Cherry, HcRed, mPlum), luminescent proteins (e.g., luciferase, β-galactosidase, chloramphenicol acetyltransferase, β-glucuronidase), secretion signal sequences (e.g., Igκ signal sequence), protease recognition sequences (e.g., TEV protease recognition sequence), expression enhancement sequences, solubilization sequences, and multimerization domains (e.g., cartilage oligomeric matrix protein domain, leucine zipper domain, collagen-like domain, cholera toxin B subunit domain, tetrabrachion coiled-coil domain, reovirus σ1 protein domain, hepatitis delta antigen domain). When the antibody of the present invention is conjugated or fused to a multimerization domain, additional antibodies of the same or different type, or their antigen-binding fragments, may further be conjugated or fused to the multimerization domain, thereby forming multimers (homomultimers or heteromultimers).

The antibody of the present invention may be chemically modified as long as the binding to CKAP4 is not significantly impaired.

In the antibody of the present invention, the C-terminus may be any of a carboxyl group (-COOH), a carboxylate (-COO-), an amide group (-CONH₂), and an ester group (-COOQ). Examples of Q in the ester group include C₁₋₆ alkyl groups such as methyl, ethyl, n-propyl, isopropyl, and n-butyl; C₃₋₈ cycloalkyl groups such as cyclopentyl and cyclohexyl; C₆₋₁₂ aryl groups such as phenyl and α-naphthyl; C₇₋₁₄ aralkyl groups such as phenyl-C₁₋₂ alkyl groups such as benzyl and phenethyl, and α-naphthyl-C₁₋₂ alkyl groups such as α-naphthylmethyl; and pivaloyloxymethyl groups.

In the antibody of the present invention, carboxyl groups (or carboxylates) other than the C-terminal carboxyl group may be amidated or esterified. In this case, examples of the ester include the C-terminal esters described above.

The antibody of the present invention further include antibodies in which the amino group of the N-terminal amino acid residue is protected with a protecting group (e.g., a C₁₋₆ acyl group such as a C₁₋₆ alkanoyl group, for example, formyl or acetyl); antibodies in which an N-terminal glutamine residue, which can be generated by in vivo cleavage, is converted to pyroglutamic acid; antibodies in which substituents on amino acid side chains (e.g., -OH, -SH, an amino group, an imidazole group, an indole group, and a guanidino group) are protected with an appropriate protecting group (e.g., a C₁₋₆ acyl group such as a C₁₋₆ alkanoyl group, for example, formyl or acetyl); and complex proteins such as so-called glycoproteins in which glycans are attached.

The antibody of the present invention also encompasses antibodies in which a drug and/or a label is linked to the antibody (conjugates). The drug is not particularly limited as long as it has physiological activity. Examples of the drug include proteins, peptides, low-molecular-weight compounds, nucleic acids, and sugar chains. Specific examples include antitumor agents described below. Examples of the label include radioactive labels and fluorescent labels.

### 2. Method for Producing Antibodies or Antigen-binding Fragments thereof

The antibody of the present invention can be produced by various methods.

The antibody of the present invention can be produced, for example, by a method comprising the steps of culturing a host transformed with a polynucleotide comprising a coding sequence of the antibody of the present invention (a polynucleotide of the present invention), and recovering a fraction containing the antibody of the present invention.

The polynucleotide of the present invention is not particularly limited as long as it comprises a coding sequence for the antibody of the present invention. Preferably, the polynucleotide of the present invention can comprise the above coding sequence in a state capable of expressing the antibody of the present invention. The polynucleotide of the present invention may contain other sequences in addition to the above coding sequence. Examples of other sequences include a secretion signal peptide coding sequence, a promoter sequence, an enhancer sequence, a repressor sequence, an insulator sequence, an origin of replication, and a drug resistance gene coding sequence, which are arranged adjacent to the antibody coding sequence of the present invention. The polynucleotide of the present invention may be a linear polynucleotide or a circular polynucleotide. In one embodiment, the polynucleotide of the present invention is an expression vector.

Specific examples of the polynucleotide of the present invention include
(I) a polynucleotide comprising a nucleotide sequence encoding at least one selected from the group consisting of heavy chains and a heavy chain variable region of the antibody of the present invention,
(II) a polynucleotide comprising a nucleotide sequence encoding at least one selected from the group consisting of light chains and a light chain variable region of the antibody of the present invention, and
(III) a polynucleotide comprising a nucleotide sequence encoding at least one selected from the group consisting of heavy chains and a heavy chain variable region of the antibody of the present invention, and a polynucleotide comprising a nucleotide sequence encoding at least one selected from the group consisting of light chains and a light chain variable region of the antibody of the present invention.

Examples of the host include, but are not limited to, prokaryotic cells and eukaryotic cells. Among these, mammalian cells, which are eukaryotic cells, such as HEK293 cells, CHO cells, NS0 cells, and SP2/O cells, are preferable from the viewpoint of more efficiently expressing antibodies. The methods of transformation, culture, and recovery are not particularly limited, and known methods used in antibody production can be used. After recovery, the antibody of the present invention may be purified as necessary. Purification can be carried out by a method known in antibody production, such as chromatography or dialysis.

Polynucleotides such as DNA and RNA may be chemically modified, as exemplified below. To prevent degradation by hydrolytic enzymes such as nucleases, the phosphate residue (phosphate) of each nucleotide can be replaced with, for example, chemically modified phosphate residues such as phosphorothioate (PS), methylphosphonate, or phosphorodithioate. Further, the 2-position hydroxy group of the sugar (ribose) of each ribonucleotide may be replaced with -OR (wherein R represents, for example, -CH₃, -CH₂CH₂OCH₃, -CH₂CH₂NHC(NH)NH₂, -CH₂CONHCH₃, or -CH₂CH₂CN). Further, the base moiety (pyrimidine, purine) may be chemically modified. Examples of such chemical modifications include introduction of a methyl group or a cationic functional group to the 5-position of the pyrimidine base, and replacement of the carbonyl group at the 2-position with a thiocarbonyl group. Examples include, but are not limited to, modifications in which the phosphate moiety or hydroxyl moiety is modified with, for example, biotin, an amino group, a lower alkylamine group, or an acetyl group.

### 3. Cells

In one embodiment, the present invention relates to a cell comprising the polynucleotide of the present invention.

The cell includes, for example, a cell into which the polynucleotide has been introduced.

The cell is preferably an isolated cell. The cell may be a cultured cell. The cultured cell may be a primary cultured cell or a passaged cultured cell. The cell may be an established cell line. The cell may be an iPS cell.

Examples of the cell include *Escherichia coli* such as *Escherichia coli* K12, *Bacillus* bacteria such as *Bacillus subtilis* MI114, yeasts such as *Saccharomyces cerevisiae* AH22, insect cells such as Sf cell lines derived from *Spodoptera frugiperda* or HighFive cell lines derived from *Trichoplusia ni*, and animal cells. Preferable examples of animal cells include cultured cells derived from mammals, and specific examples include COS7 cells, CHO cells, HEK293 cells, HEK293FT cells, HeLa cells, PC12 cells, N1E-115 cells, and SH-SY5Y cells.

### 4. Applications

In one embodiment, the present invention relates to a pharmaceutical composition, a reagent, an antitumor agent, an antitumor immune response activator, and the like, containing the antibody of the present invention (which may be referred to below as "active ingredient").

### Application

Since CKAP4 is highly expressed in cancer cells and promotes the proliferation of cancer cells as disclosed in PTL 1, the antitumor agent and antitumor immune response activator of the present invention suppress the expression or function of CKAP4 to thereby achieve the suppression of cancer cell proliferation and the activation of antitumor immune response. Therefore, the antitumor agent or the antitumor immune response activator of the present invention can be used to prevent or treat cancer. When the antibody of the present invention is used as an antitumor agent or an antitumor immune response activator, examples of the target cancer include, but are not limited to, solid cancers such as lung cancer, pancreatic cancer, esophageal cancer, colorectal cancer, colon cancer, gastric cancer, rectal cancer, liver cancer, breast cancer, bladder cancer, prostate cancer, cervical cancer, head and neck cancer, bile duct cancer, gallbladder cancer, oral cancer, tongue cancer, pharyngeal cancer, laryngeal cancer, brain tumor, glioma, glioblastoma, hepatoblastoma, glioblastoma multiforme, and peritoneal dissemination; and hematological cancers such as leukemia and malignant lymphoma.

Further, as disclosed in PTL 1, anti-CKAP4 antibodies effectively exert an inhibitory effect on the proliferation of cancer cells expressing CKAP4, in particular, cancer cells expressing both CKAP4 and DKK1. Therefore, cancers expressing CKAP4, in particular, cancers expressing both CKAP4 and DKK1, are considered to be suitable targets to which the antibody of the present invention is applied. Among cancers, lung cancer, pancreatic cancer, esophageal cancer, liver cancer, and hepatoblastoma frequently exhibit high expression of CKAP4 and DKK1, thus being particularly suitable as cancers to which the antibody of the present invention is applied.

The expression of CKAP4 in cancer can be confirmed by performing immunohistochemical staining on collected cancer tissue. Specifically, the collected cancer tissue is immunostained using an anti-CKAP4 antibody, and if regions where CKAP4 expression is observed account for 5% or more of the tumor area, CKAP4 is determined to be expressed. Preferable examples of cancers to which the antibody of the present invention is applied include cancers in which the CKAP4 expression region accounts for 5% or more of the tumor area, more preferably cancers in which the CKAP4 expression region accounts for 20% or more of the tumor area, and particularly preferably cancers in which the CKAP4 expression region accounts for 50% or more of the tumor area.

The expression of CKAP4 in cancer can be confirmed by recovering RNA from collected cancer tissue or body fluids such as serum (test sample) and measuring the RNA by quantitative PCR or by immunostaining the cancer tissue. Alternatively, CKAP4 level in serum can also be measured by ELISA. In this case, whether CKAP4 is expressed can be determined by using non-cancerous tissue or non-cancerous patient's body fluid from the same subject (control sample) as a reference. Specifically, when the amount of CKAP4 in a test sample (for example, immunostaining of cancer tissue) is greater than the amount of CKAP4 in a control sample (for example, a cell lysate of non-cancerous tissue from the same subject), it is determined that CKAP4 is highly expressed in the cancer. Further, when a body fluid is used, it can be used after being subjected to purification (for example, by concentrating extracellular vesicles, such as exosomes), in view of further enhancing sensitivity.

The expression of CKAP4 in lung adenocarcinoma can be evaluated, for example, based on specific numerical values. That is, when serum CKAP4 levels were measured by sandwich ELISA using the mouse anti-CKAP4 antibodies, approximately 25% of immunohistologically CKAP4-positive cases were positive for serum CKAP4 levels (0.1 ng/ml or higher), whereas 4.5% of CKAP4-negative cases were positive for serum CKAP4 levels. Thus, the results suggest that CKAP4 is highly expressed in cases with positive serum CKAP4 levels.

The expression of CKAP4 in pancreatic cancer can be evaluated based on specific numerical values, for example, as follows. That is, when serum CKAP4 levels were measured by sandwich ELISA method using the mouse anti-CKAP4 antibodies, approximately 64% of immunohistologically CKAP4-positive cases were positive for serum CKAP4 levels (1 ng/ml or higher), whereas none of the CKAP4-negative cases were positive for serum CKAP4 levels, thus suggesting that CKAP4 is highly expressed in cases with positive serum CKAP4 levels.

Confirmation of DKK1 expression in cancer can also be performed in the same manner as in the case of CKAP4. DKK1 expression can be confirmed, for example, by a method of immunostaining collected cancer tissue, or a method of recovering RNA from collected cancer tissue and measuring by quantitative PCR. Specifically, for example, when a collected cancer tissue is immunostained using an anti-DKK1 antibody, if a region where DKK1 expression is observed accounts for 5% or more of the tumor area, it is determined that DKK1 is expressed. Preferred examples of cancers to which the antibody of the present invention is applied include cancers in which DKK1 expression regions account for 5% or more of the tumor area, more preferably cancers in which DKK1 expression regions account for 20% or higher of the tumor area, and particularly preferably cancers in which DKK1 expression regions account for 50% or more of the tumor area.

When RNA is recovered from collected cancer tissue and DKK1 is measured, if the amount of DKK1 in the cell lysate of the cancer tissue is greater than the amount of DKK1 in the cell lysate of non-cancer tissue from the same subject, it is determined that DKK1 is expressed in the cancer.

### Administration Form

As regards the administration form of the antibody of the present invention, as long as antitumor activity is obtained, the antibody of the present invention can be administered parenterally. Specific examples of the administration form of the antibody of the present invention include injection administration (e.g., intravenous injection, subcutaneous injection, intramuscular injection, intraperitoneal injection, and local injection into the affected area).

The dosage of the antibody of the present invention may be appropriately set according to, for example, the administration form, the type of cancer to be treated, and the degree of the patient's symptoms. For example, as a single dose of the antibody of the present invention, about 0.1 mg to 20 mg/kg body weight may be usually administered at a frequency of about once every 1 to 12 weeks.

The antibody of the present invention may be used alone, or may be used in a combination with one or more other drugs having antitumor activity and/or radiation therapy. Examples of other drugs used in combination with the antibody of the present invention include antitumor agents used in chemotherapy. Specific examples of such antitumor agents include antimetabolites, alkylating agents, microtubule-acting agents, anticancer antibiotics, topoisomerase inhibitors, platinum agents, and kinase inhibitors. Among these, gemcitabine, paclitaxel, lenvatinib, and the like are particularly preferred.

### Dosage Form

The pharmaceutical composition of the present invention, the reagent of the present invention, the antitumor agent of the present invention, and the antitumor immune response activator of the present invention are prepared in a dosage form according to the administration form. Examples of the dosage form of the antitumor agent and antitumor immune response activator of the present invention include liquid preparations such as solutions, suspensions, emulsions, and injections.

The pharmaceutical composition of the present invention, the reagent of the present invention, the antitumor agent of the present invention and the antitumor immune response activator of the present invention are formulated by adding pharmaceutically acceptable carriers and additives according to their dosage form. For example, in the case of a liquid preparation, the antitumor agent of the present invention and the antitumor immune response activator of the present invention can be formulated using physiological saline, buffer, or the like.

The reagent of the present invention may be in the form of a kit containing an active ingredient. The kit may contain instruments, reagents, and the like. Examples of such instruments and reagents include test tubes, microtiter plates, purification columns, labeled antibodies, standard samples (positive controls, negative controls), and the like.

### Examples

The present invention is described in detail below based on Examples. However, the present invention is not limited by these Examples.

### 1. Test method

### 1-1. Preparation of Mouse Anti-CKAP4 Antibody (3F11-2B10)

Mouse anti-CKAP4 antibody was prepared as follows. Specifically, hybridomas expressing mouse anti-CKAP4 antibody 3F11-2B10 cultured in 10% FCS RPMI-1640 medium containing recombinant human IL-6 protein (1 ng/ml, R&D) in 100 mm diameter culture dishes were collected, suspended in ice-cold PBS (1 x 10⁷ cells suspended in 100 µl PBS), and kept on ice until their transplantation into mice. The hybridomas were then intraperitoneally transplanted into 6- to 8-week-old male BALB/cAnNCrj-nu immunodeficient mice. After abdominal distension was observed, the mice were euthanized by carbon dioxide treatment, and ascites fluid was collected by puncturing the abdominal cavity. After stepwise centrifuge (1st time: 2,000 x g for 10 minutes, 2nd time: 10,000 x g for 10 minutes, 3rd time: 100,000 x g for 10 minutes), the supernatant was subjected to antibody purification. Protein A-R28 sepharose (bed volume 1 ml: Ab-Capcher, ProteNova No. P-002-10) was added to the supernatant and rotated and mixed at 4°C for 12 hours. The beads were washed 3 times with 10 ml of ice-cold PBS, transferred to a poly-prep column (Bio-Rad No. 7311550), and 3F11-2B10 was eluted with 10 ml of 0.1 M glycine (pH 2.8). Ten fractions (1 ml each) were collected, and each fraction was neutralized with 100 µl of 1 M Tris-HCl (pH 7.5). The fractions containing antibody were dialyzed against ice-cold PBS.

### 1-2. cDNA Cloning of Mouse Anti-Human CKAP4 Antibody

Total RNA was isolated from 3F11-2B10 hybridoma cells using the RNeasy Mini Kit (QIAGEN), and cDNA was synthesized using the SMARTer RACE cDNA Amplification Kit (CLONTECH Laboratories). The cDNAs encoding the heavy chain variable region and light chain variable region were amplified by the 5'-RACE method using primers specifically designed for the constant regions of IgG2b heavy chain and κ light chain, using TaKaRa Bio's PCR reagents (PrimeSTAR Max for the heavy chain variable region and PrimeSTAR HS for the light chain variable region).

All mouse anti-CKAP4 antibodies used in the Examples were expressed by introducing the expression vectors constructed as described above into Expi293F cells or FreeStyle 293F cells (Thermo Fisher Scientific) and expressing the antibodies according to the manufacturer's instructions. After expression, the mouse anti-CKAP4 antibodies were purified from the conditioned medium by Ab-Capcher resin (ProteNova) followed by dialysis using 50 mM sodium acetate (pH 4.5). The anti-KLH antibody was purified using MabSelect SuRe resin (Cytiva), followed by gel filtration chromatography with one of the buffers describe below (D-PBS(-) or 20 mM histidine, 150 mM NaCl, pH 6.0). All purified antibodies were sterile filtered and stored at -80°C.

### 1-3. Preparation and Purification of Humanized Antibodies

Complementarity determining regions (CDRs) were defined by the Kabat method, IMGT method, and Paratome method. The most similar human germline sequences were identified using the IMGT/DomainGapAlign database. The CDRs from Kabat/IMGT/Paratome were combined and introduced into the human germline framework sequences.

To purify 10 mg of humanized anti-CKAP4 antibody (Hv1Lt1), suspended CHO cells (5 × 10⁸ cells) were transfected with pCEC4.2-3F11-2B10 hVHv1hIgG CH and pCEC4.2-3F11-2B10 hVLv1hIgK CL, and cultured for 7 days in CH200 medium supplemented with L-alanyl-L-glutamine. Cells were pelleted by centrifugation at 12,000 × g for 10 minutes, and the supernatant was collected. The supernatant (approximately 500 ml) was added to an Ab-Capture TM (Protein A mutant) column, and the column was washed 5 times with 5 ml of 1 x TBS. The antibody was then eluted with 7-10 ml of Pierce^{™} Gentle Ag/Ab Elution Buffer at pH 6.6, dialyzed (pore size: 40 to 50Å) against TBS and PBS, and stored at -80°C until use.

### 1-4. Measurement of the Binding Affinity of Anti-CKAP4 Antibodies by the Surface Plasmon Resonance (SPR) Method

For the measurement, a Biacore^{TM} T200 system (GE Healthcare Bio-Sciences AB, Uppsala, Sweden) pre-equilibrated at 25°C was used. After 10 µg/ml of humanized anti-CKAP4 antibody and mouse anti-CKAP4 antibody were captured on an anti-human IgG- or anti-mouse IgG-immobilized Biacore Series S sensor chip CM5 (GE Healthcare Bio-Sciences AB) for 2 minutes and serially diluted human CKAP4 extracellular region (465-602 amino acids) was injected at a flow rate of 10 µl/min for 2 minutes, a buffer was allowed to flow in for 2 minutes. Sensorgrams were fitted to a global 1:1 Langmuir binding model using Biacore T200 Evaluation Software (GE Healthcare Life Sciences, Chicago, IL, USA). The binding affinity (using the equilibrium dissociation constant [KD]) was calculated by dividing the dissociation rate constant by the association rate constant (kd/ka).

### 1-5. Cells and Materials

S2-CP8 pancreatic cancer cells were purchased from the Cell Resource Center for Biomedical Research, Institute of Development, Aging and Cancer, Tohoku University. Hep3B cells were purchased from the American Type Culture Collection (ATCC) (Rockville, MD, USA). HuH-7 cells were purchased from the Japanese Collection of Research Bioresources. TE-5 and TE-8 cells were obtained from the RIKEN BioResource Center Cell Bank. S2-CP8, MIA PaCa-2, HuH-7, HeLa S3, and MDCK I cells were maintained in DMEM. TE-5 and TE-8 were maintained in RPMI-1640 containing 10% fetal bovine serum (FBS). Hep3B cells were maintained in E-MEM (FUJIFILM Wako Pure Chemical Corporation; catalog number 051-07615). All cells were authenticated in July 2022 using short tandem repeat analysis and mycoplasma testing (MycoStrip, InvivoGen).

Lenvatinib (mesylate) was obtained from ChemScene (NJ, USA) and dissolved in a 0.5% (wt/vol) methylcellulose 400 solution (FUJIFILM Wako Pure Chemical Corporation) for in vivo experiments in mice. Nanoparticle albumin-bound paclitaxel (nab-PTX; Abraxane) was obtained from Taiho Pharmaceutical Co., Ltd. and dissolved in physiological saline for in vivo experiments in mice. Coomassie Brilliant Blue staining was performed using Bullet CBB Stain One (Nacalai Tesque, Inc.). Other materials and chemicals used were commercially available products.

### 1-6. Animal Experiments

Mice were housed in a well ventilated, standardized environment with a 12:12 hour light-dark cycle and a free access to food and water. Sample sizes were selected to be optimized as the maximum number of samples per independent experiment, while considering the 3Rs (reduction/refinement/replacement) of animal experiments.

### 1-7. Xenograft Tumor Formation Assay

The xenograft tumor formation assay was performed using a modified version of a previously reported method. BALB/cAJcl-nu/nu mice (8-week-old male nude mice; CLEA Japan) were anesthetized with a combination of medetomidine (0.3 mg/kg body weight), midazolam (4 mg/kg), and butorphanol (5 mg/kg). Subsequently, a suspension of S2-CP8 (2.5 × 10⁶ cells) or MIA PaCa-2 (5 × 10⁶ cells) in 120 µl PBS or a suspension of Hep3B cells (5 × 10⁶ cells) in 150 µl Matrigel was injected subcutaneously into both flanks of mice, and antibody administration was started when the tumor volume had reached 50 mm³.

To investigate the antitumor effect of anti-CKAP4 antibody, anti-CKAP4 antibody (3F11-2B10, silent Fc antibody, or humanized antibody Hv1Lt1, 200-400 µg/dose) or control IgG was injected intraperitoneally or intravenously twice a week. Tumor formation was continued for an additional 21 days (S2-CP8 cells or Hep3B cells) or 35 days (MIA PaCa-2 cells). Xenograft tumor volume <50 mm³ at the start of treatment was excluded from the analysis. The excised tumors were measured and weighed. Tumor volume was calculated by the following formula: $\left(Length\right) \times \left(Width\right) \times \left(Width\right) / 2 .$

### 1-8. Combination Therapy Assay of Humanized Anti-CKAP4 Antibody and Other Therapeutic Agents

To investigate the antitumor effects of combined use of humanized anti-CKAP4 antibody Hv1Lt1 with other drugs such as gemcitabine, nanoparticle albumin-based paclitaxel (nab-PTX), and lenvatinib, mice subcutaneously transplanted with S2-CP8 cells or Hep3B cells were randomly divided into 4 groups (control IgG + PBS or saline; Hv1Lt1 + PBS or saline; control IgG + drug; and Hv1Lt1 + drug). Hv1Lt1 antibody or control IgG (280 µg/dose) was intraperitoneally administered twice a week. Gemcitabine (100 µg/dose, once weekly) or nab-PTX (10 mg/kg, twice weekly) was intraperitoneally administered. While lenvatinib (1 mg/kg) or a 0.5% (wt/vol) methylcellulose 400 solution was orally administered once a day, 6 days per week, tumor formation was continued for an additional 21 days, after which the mice were euthanized. The volume and weight of the excised tumors were measured.

### 1-9. In vivo Toxicity Evaluation of Humanized Anti-CKAP4 antibody Hv1Lt1

To evaluate the in vivo toxicity of long-term administration of humanized anti-CKAP4 antibody, Hv1Lt1 or control IgG (200 µg/injection) was intraperitoneally injected to C57BL/6 mice (8-week-old male nude mice; CLEA Japan) twice a week for 3 months. Subsequently, the body weight of the mice was measured, and blood samples (whole blood and serum) were collected. Further, heart, lung, liver, stomach, small intestine, spleen, pancreas, and kidney were collected for histological evaluation. Blood tests were outsourced to Oriental Yeast.

### 1-10. Histological Analysis

Organs excised for histological analysis were fixed in 10% formalin and embedded in paraffin. 5 µm sections were stained with hematoxylin and eosin (H&E) according to standard protocols. Bright-field images were obtained using an automated imaging platform (NanoZoomer) (Hamamatsu Photonics, Japan).

### 1-11. Immunoprecipitation Assay

The immunoprecipitation assay was performed as previously reported. Cells (60 mm diameter dish) were lysed in 500 µl of Nonidet P-40 (NP-40) buffer (20 mM Tris-HCl (pH 8.0), 10% glycerol, 137 mM NaCl, 1% NP-40) supplemented with protease inhibitors (10 µg/ml leupeptin, 20 µg/ml aprotinin, and 1 mM phenylmethanesulfonyl fluoride). After centrifugation, the solubilized material was mixed with the primary antibody and 50% slurry of 40 µl protein G-Sepharose beads (GE Healthcare Bio-Sciences, Buckinghamshire, UK) at 4°C for 1 hour. After the precipitates were washed with NP 40 buffer 3 times and then detected using a labeled antibody.

### 1-12. Immunocytochemical Analysis

For fluorescence immunostaining, cultured cells were fixed with PBS containing 4% paraformaldehyde (PFA) and permeabilized for 10 minutes with PBS containing 0.2% (wt/vol) Triton X-100 and 2 mg/ml bovine serum albumin (BSA). Cells were blocked with blocking buffer (PBS containing 2 mg/ml BSA). The cells were then mixed with the primary antibody diluted in PBS for 1 hour or overnight, washed 3 times with PBS, and then stained for 1 hour with the secondary antibody diluted in PBS (Alexa Fluor 488-bond, Invitrogen). The stained cells were observed using an LSM810 laser scanning microscope. When CKAP4 protein localized on the cell surface is immunostained, S2-CP8 cells were mixed with anti-CKAP4 antibody for 1 hour under non-permeabilized conditions. After washing, the cells were fixed and stained with a secondary antibody. For immunohistochemical staining, after tumor masses excised from animals were fixed in PBS containing 4% paraformaldehyde (PFA) and paraffin blocks were prepared, tissue sections with a thickness of 5 µm were prepared. After deparaffinization of tissue sections, antigen retrieval was performed by treatment in EDTA buffer (pH 9) at 110°C for 15 minutes. The tissue sections were blocked with a G-block solution (Genostaff) to activate endogenous HRP, then treated with the primary antibody for 1 hour, washed, and then treated with the HRP polymer-labeled secondary antibody for 1 hour. After the target antigen was stained with 3,3'-diaminobenzidine (DAB) substrate, cell nuclei were stained with a hematoxylin solution and observed using an optical microscope.

### 1-13. Two-dimensional (2D) Cell Proliferation Assay

The cell proliferation assay was performed using the CyQUANT NF Cell Proliferation Assay Kit (Thermo Fisher Scientific) according to the manufacturer's protocol. The relative fluorescence intensity of the counted cells was expressed in arbitrary units.

To examine the cytotoxic effect of the humanized anti-CKAP4 antibody, HeLa S3 cells (1 × 10³ cells) were seeded in a 96-well plate, 200 µl of DMEM containing 10% FBS and 10 µg/ml Hv1Lt1 or control IgG was added, and the cells were cultured for 5 days. Cell proliferation ability was measured in the same matter as described above.

### 1-14. Three-dimensional (3D) Cell Proliferation Assay

Three-dimensional Matrigel culture assays were performed with modifications to a previously reported method. S2-CP8 cells (2 x 10⁴ cells) were suspended in DMEM containing 0.1% bovine serum albumin and 2% Matrigel (BD Biosciences, San Jose, CA), and 20 µg/ml anti-CKAP4 antibody or control IgG was added, followed by culture on solidified Matrigel for 5 to 7 days.

### 1-15. DKK1-CKAP4 Binding Assay In Vitro

The DKK1-CKAP4 bond assay was performed with modifications to a previously reported method. To investigate the effect of anti-CKAP4 antibody on the binding of DKK1 to CKAP4, various concentrations (0-3 µg/ml) of anti-CKAP4 antibody were mixed with a 0.5 nM GST-CKAP4-Extracellular domain (ECD) in 500 µl of NP40 buffer while rotating at 4°C for 1 hour. After the resulting mixture was mixed with 2.5 nM DKK1-FLAG for 1 hour, GST-CKAP4-ECD was precipitated with glutathione Sepharose and then washed with NP40 buffer 3 times. The precipitate was detected with an anti-DKK1 antibody and analyzed using ImageJ software (National Institutes of Health, Bethesda, MD).

### 1-16. Quantitative PCR

Quantitative PCR was performed according to a previous report.

### 1-17. Establishment of Organoid Culture from Pancreatic Cancer Model Mice

Primary pancreatic ductal adenocarcinoma tissue was obtained from genetically modified pancreatic cancer model mice, which are a KPC model (a mouse model that develops pancreatic cancer with the genotype LSL-KrasG12D/+; Trp53fl/fl; Elastase-Cre) provided by the Department of Gastroenterology and Hepatology, Osaka University. Fresh tumor tissue obtained from the KPC model was minced to obtain cells, which were then dissociated in DMEM/F12 medium containing 0.25% Trypsin-EDTA and 100 U DNase I (Sigma, D4527) with stirring at 37°C for 30 minutes. After passing the cells through a 40 mm sterile filter (BD Biosciences, 352340), 5,000 cells were suspended in 20 mL of ice-cold Matrigel solution. Pancreatic cancer cells embedded in Matrigel were placed as droplets into a 24-well tissue culture plate, allowed to solidify by incubating at 37°C for 20minutes, and then cultured in complete growth medium. The established mouse pancreatic cancer organoids (KPC organoids) were passaged every week. Complete growth medium was prepared at the time of use. The composition is as follows: DMEM/F12, 1 x N-2 (Life Technologies, 17502048), 1 x B-27 Supplements (Life Technologies, 17504044), 10 mmol/L HEPES/NaOH (pH 7.4), 50 ng/mL EGF (R&D Systems, 236-EG), 100 ng/mL Noggin (R&D Systems, 1967-NG), 100 ng/mL R-Spondin 1 (R&D Systems, 7150-RS), 100 ng/mL FGF10 (Pepro-Tech, 100-26), 10 ng/mL Gastrin I (Sigma, G9145), 10 mmol/L Y27632 (WAKO, 253-00513), 500 mM N-acetylcysteine (Sigma, A9165), 10 mM Nicotinamide (Sigma, N0636), and 500 nM A83-01 (Tocris, #2939).

### 1-18. Orthotopic Transplantation of KPC Organoids into Syngeneic Immunodeficient Mice

5 x 10⁴ single cells prepared from KPC organoids stably expressing human CKAP4 and human DKK1 (KPC/CKAP4/DKK1) were suspended in 50 µl of Matrigel and stored on ice until transplantation. A 1.5 cm incision was made in the left dorsal flank region of a C57BL/6 mouse, and the spleen was exposed from the abdominal cavity. The spleen was fixed with sterile cotton, and the pancreas beneath the spleen was exposed. After the Matrigel cell suspension was injected into the tail of the pancreas using a 29G syringe and the pancreas and spleen were returned to the abdominal cavity, the incision was sutured. The size of the transplanted organoids was measured every week by ultrasound examination. When tumor lesions were detected, the mice were randomly divided into two groups and intraperitoneal administration of humanized anti-CKAP4 antibody Hv1Lt1 (280 µg/dose, twice a week) or control IgG was initiated. After 56 days, the mice were euthanized, tumors located in the tail of the pancreas were excised, and their weight was measured. Tumor volume was calculated using the following formula: $\left(Length\right) \times \left(Width\right) \times \left(Width\right) / 2$ [Mitsuishi, 2012 #4024].

### 1-19. RNA Sequencing

Bulk RNA-sequence analysis was performed on RNA samples excised from pancreatic tumors in which KPC organoids were orthotopically transplanted into immunodeficient mouse models (Fig. 4D). Four control tumors and four Hv1Lt1-treated tumors were randomly selected from each group. The library was prepared using the TruSeq stranded mRNA sample prep kit (Illumina) according to the manufacturer's instructions. The mRNA sample underwent whole transcriptome sequencing using the DNBSEQ sequencing platform in 100 base pair end mode. Sequenced reads were preprocessed with Trim Galore! v0.6.7 and quantified with Salmon v1.4.0 using gcBias and validateMappings flags. GENCODE vM26 (GRCm39) annotation was used as the reference for transcripts. All procedures were implemented using the RNA-sequence pipeline ikra v2.0.1 (http://doi.org/10.5281/zenodo.4718200) with default parameters. The quantified transcript-level scaled TPM was summarized to gene-level scaled TPM using tximport v1.22.0. Downstream pathway analysis was performed using iDEP2.0 (BMC Bioinformatics 2018; 19: 534, doi:10.1186/s12859-018-2486-6). After removing low-expression genes with less than 0.5 counts per million (CPM) in at least one sample, 15,238 genes out of 22,216 genes from 8 samples were selected as candidates. After normalization with VST, main component analysis was performed. Hierarchical clustering was performed on the top 2000 genes from the perspective of standard deviation. Differential expression analysis by DESeq2 was performed in iDEP using the following parameters; false discovery rate <0.1, minimum fold change >1.5.

### 1-20. Statistical Analysis

Student's *t*-test or the Mann-Whitney U test was used to determine statistical differences between the average value of two groups. Analysis of variance (ANOVA) with Dunnett's post-hoc test was used to compare average value among three or more groups. P < 0.05 was considered statistically significant. Statistical analyses were performed using GraphPad Prism 9 (GraphPad Software, La Jolla, CA, USA) or JMP software (SAS Institute. Inc., Cary, NC, USA).

### 2. Results

### 2-1. cDNA Cloning and Purification of Mouse Anti-CKAP4 Antibody 3F11-2B10

The cDNA was synthesized using RACE cDNA Amplification technology [PMID: 2461560] based on total RNA isolated from 3F11-2B10 hybridoma cells (PTL 1). The base sequence of the cloned heavy chain variable region and light chain variable region of 3F11-2B10 were determined, and the cDNA contained open reading frames encoding proteins of 141 and 127 amino acids.

mF18 (mIgG1-based) and mFa31 (mIgG2a-based) were prepared as 2 types of silent Fc mouse anti-CKAP4 antibodies created by introducing mutations including P235K/S239K into the Fc domain, which is the Fc receptor binding site. The mutations in mouse IgG1-type silent Fc (mF18) were P235K/S239K, and the mutations in mouse IgG2-type silent Fc (mFa31) were L235R/G236R/S239K. The molecular weights of these recombinant antibodies were similar to those of the original antibodies under reducing conditions.

### 2-2. Analysis of Characterization of Silent Fc Mouse Anti-CKAP4 Antibody

Similar to the original mouse anti-CKAP4 antibody 3F11-2B10, the silent Fc mouse anti-CKAP4 antibody detected and immunoprecipitated human endogenous CKAP4 from S2-CP8 cell lysates (Fig. 1A). Both the silent Fc mouse anti-CKAP4 antibodies inhibited the binding of DKK1 and CKAP4 in a dose-dependent manner in vitro (Fig. 1B). When cancer cell lines (cells in which DKK1-CKAP4 signal pathway was activated), including pancreatic cancer cells S2-CP8, hepatocellular carcinoma cells (HuH7 and Hep3B), and esophageal squamous cell carcinoma cells (TE-5 and TE-8), were treated with the silent Fc mouse anti-CKAP4 antibody, AKT activity was inhibited (Fig. 1C). Tumor sphere formation of S2-CP8 cells and Huh7 cells was also inhibited by the silent Fc mouse anti-CKAP4 antibody (Fig. 1D).

Further, the silent Fc mouse anti-CKAP4 antibody (mF18) suppressed tumor formation of xenografts induced by S2-CP8 cells (Fig. 1E). Since the silent Fc mouse anti-CKAP4 antibody lacks the Fc receptor binding site and complement binding site necessary for antibody-dependent cellular cytotoxicity (ADCC) and complement-dependent cytotoxicity (CDC), these results suggest that the antitumor effect and pharmacological activity in vivo are not mediated through ADCC activity, but rather by inhibiting the binding of DKK1 to CKAP4.

### 2-3. Preparation of Humanized Anti-CKAP4 Antibody

Complementarity determining regions (CDRs) were defined by the Kabat method, IMGT method, and Paratome method. Table 1 and Table 2 show the base sequence and amino acids sequence of the variable region of the 3F11-2B10 antibody. In the table, CDRs determined by the Kabat method, IMGT method, and Paratome method, respectively, are shown.

**Table 1**

| |
|---|
| 3F11-2B10_VH (mouse) |
| (Underline: Kabat. *Italic : IMGT,* Frame: Paratome |
| • Heavy chain signal peptide code sequence |
| ATGGGATGGAGCTGTATCATCCTCTTTCTGGTAGCAGCAGCTACAGGTGTCCACTC C (SEQ ID NO: 26) |
| • Heavy chain variable region base sequence |
| CAGGTCCAATTGCAGCAGCAGCCTGGGACTGAACTGGTGAAGCCTGGGGCTTCAG TGAAGCTGTCTTGCAAGGCCTCTGGCTTCACCTTCACCAGCTACTGGATGCACTGG GTAAAACAGAGGCCTGGACAAGGCCTTGAGTGGATTGGAAATATTAATCCTAGCAA TGGTGGTACTAACTACAATGCGAAGTTCAAGAGTAAGGCCACACTGACTGTAGACA AATCCTCCAGTACAGCCTACATGCAACTCAGCAGCCTGACATCTGAGGACTCTGCG GTCTATTATTGTGCAAGATGGGTCTCCTTTAGTTACCGAGGGGCTATGGACTACTG GGGTCAAGGAACCTCAGTCACCGTCTCCTCA (SEQ ID NO: 21) |
| • Heavy chain signal peptide amino acid sequence |
| MGWSCIILFLVAAATGVHS (SEQ ID NO: 27) |
| • Heavy chain variable region amino acid sequence |
| QVQLQQPGTELVKPGASVKLSCKASGFTFTSYWMHWVKQRPGQGLEWIGNINPS NGGTNYNAKFKSKATLTVDKSSSTAYMQLSSLTSEDSAVYYCARWVSFSYRGAMDY WGQGTSVTVSS (SEQ ID NO: 22) |

**Table 2**

| |
|---|
| 3F11-2B10_VL (mouse) |
| ( Underline: Kabat. *Italic : IMGT,* 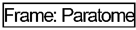 |
| • Light chain signal peptide code sequence |
| ATGGTTTTCACACCTCAGATACTTGGACTTATGCTTTTTTGGATTTCAGCCTCCAGA GGT (SEQ ID NO: 28) |
| • Light chain variable region base sequence |
| GATATTGTGCTAACTCAGTCTCCAGCCATTCTGTCTGTGACTCCAGGAGATGGCGT CAGTCTTTCCTGCAGGGCCAGTCGAGCTATTAGGAACAACCTACACTGGTTTCAAC AAAAATCACATGAGTCTCCAAGGCTTCTCATCAAGTATGTTTCCCAGTCCATCTCTG GGATTCCCTCCAGGTTCAGTGGCAGTGGATCAGGGACAGATTTCACTCTCAGTGTC GACAGTGTAGAGACTGAAGATTTTGGAGTGTATTTCTGTCAACAGAGTGACAGCTG GCCTTACACGTTCGGAGGGGGGACCAAACTGGAAATAAAA (SEQ ID NO: 23) |
| • Light chain signal peptide amino acid sequence: |
| MVFTPQILGLMLFWISASRG (SEQ ID NO: 29) |
| • Light chain variable region amino acid sequence: |
| DIVLTQSPAILSVTPGDGVSLSCRASRAIRNNLHWFQQKSHESPRLLIKYVSQSISGIP SRFSGSGSGTDFTLSVDSVETEDFGVYFCQQSDSWPYTFGGGTKLEIK (SEQ ID NO: 24) |

The identification of the most similar human germline sequence was determined by the IMGT/DomainGapAlign database. The CDRs identified by the three method were subtly different. Therefore, when humanized antibodies were produced, a region covering all CDRs determined by the three CDR determination methods (referred to as broad CDR, H₁) and a region overlapping in the three CDR determination methods (referred to as narrow CDR, H₂) were determined. Based on these two CDR patterns, humanization was carried out. The framework used in this Example was created by selecting human germline antibody sequences from a database that were similar to the mouse 3F11-2B10 sequence, and then grafting a combination of Kabat, IMGT, and Paratome CDRs onto the selected human germline framework sequence. The variant with broader CDRs was designated as v1, and the one with narrower CDRs was designated as v2.

Tables 3 to 5 show the amino acids sequences of the two humanized antibodies (Hv1Lt1 and Hv2Lt1) obtained. Table 3 shows the heavy chain amino acid sequence of Hv1Lt1. Table 4 shows the heavy chain amino acid sequence of Hv2Lt1. Table 5 shows the light chain amino acid sequence of Hv1Lt1 and Hv2Lt1 (the light chains of Hv1Lt1 and Hv2Lt1 have the same sequence).

**Table 3**

| |
|---|
| • Hv1Lt1 Heavy chain CDR1: |
| GFTFTSYWMH (SEQ ID NO: 1) |
| • Hv1Lt1 Heavy chain CDR2: |
| WIGNINPSNGGTNYNAKFKS (SEQ ID NO: 2) |
| • Hv1Lt1 Heavy chain CDR3: |
| ARWVSFSYRGAMDY (SEQ ID NO: 3) |
| • Hv1Lt1 Heavy chain signal peptide amino acid sequence: |
| MDWTWRVFCLLAVAPGAHS (SEQ ID NO: 30) |
| • Hv1Lt1 Heavy chain variable region: |
| QVQLVQSGAEVKKPGASVKVSCKASGFTFTSYWMHWVRQAPGQGLEWIGNINPSN GGTNYNAKFKSRVTMTRDTSTSTVYMELSSLRSEDTAVYYCARWVSFSYRGAMDY WGQGTLVTVSS (SEQ ID NO: 4) |
| • Hv1Lt1 Heavy chain constant region: |
| ASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPA PELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQP REPQVYTLPPSRDELTKNQVSLTCLVKGFYPSDIAVEWESNGQPENNYKTTPPVLDS DGSFFLYSKLTVDKSRWQQGNVFSCSVMHEALHNHYTQKSLSLSP (SEQ ID NO: 16) |
| • Hv1Lt1 Heavy chain: |
| MDWTWRVFCLLAVAPGAHSQVQLVQSGAEVKKPGASVKVSCKASGFTFTSYWMH WVRQAPGQGLEWIGNINPSNGGTNYNAKFKSRVTMTRDTSTSTVYMELSSLRSEDT AVYYCARWVSFSYRGAMDYWGQGTLVTVSSASTKGPSVFPLAPSSKSTSGGTAALG CLVKDYFPEPVTVSWNSGALTSGVHTFPAVLQSSGLYSLSSVVTVPSSSLGTQTYICN VNHKPSNTKVDKKVEPKSCDKTHTCPPCPAPELLGGPSVFLFPPKPKDTLMISRTP EVTCVVVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQQGNVFSC SVMHEALHNHYTQKSLSLSP (SEQ ID NO: 5) |

**Table 4**

| |
|---|
| • Hv2Lt1 Heavy chain CDR1: |
| SYWMH (SEQ ID NO: 6) |
| • Hv2Lt1 Heavy chain CDR2: |
| INPSNGGT (SEQ ID NO: 7) |
| • Hv2Lt1 Heavy chain CDR3: |
| WVSFSYRGAMDY (SEQ ID NO: 8) |
| • Hv2Lt1 : Heavy chain single peptide amino acid sequence |
| MDWTWRVFCLLAVAPGAHS (SEQ ID NO: 30) |
| • Hv2Lt1 Heavy chain variable region |
| QVQLVQSGAEVKKPGASVKVSCKASGYTFTSYWMHWVRQAPGQGLEWMGIINPSN GGTSYAQKFQGRVTMTRDTSTSTVYMELSSLRSEDTAVYYCARWVSFSYRGAMDYW GQGTLVTVSS (SEQ ID NO: 9) |
| • Hv2Lt1 Heavy chain constant region |
| ASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPA PELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQP REPQVYTLPPSRDELTKNQVSLTCLVKGFYPSDIAVEWESNGQPENNYKTTPPVLDS DGSFFLYSKLTVDKSRWQQGNVFSCSVMHEALHNHYTQKSLSLSP (SEQ ID NO: 16) |
| • Hv2Lt1 Heavy chain: |
| MDWTWRVFCLLAVAPGAHSQVQLVQSGAEVKKPGASVKVSCKASGYTFTSYWMH WVRQAPGQGLEWMGIINPSNGGTSYAQKFQGRVTMTRDTSTSTVYMELSSLRSEDT AVYYCARWVSFSYRGAMDYWGQGTLVTVSSASTKGPSVFPLAPSSKSTSGGTAALG CLVKDYFPEPVTVSWNSGALTSGVHTFPAVLQSSGLYSLSSVVTVPSSSLGTQTYICN VNHKPSNTKVDKKVEPKSCDKTHTCPPCPAPELLGGPSVFLFPPKPKDTLMISRTP EVTCVVVDVSHEDPEVKFNWYVDGVEVHNAKTKPREEQYNSTYRVVSVLTVLHQD WLNGKEYKCKVSNKALPAPIEKTISKAKGQPREPQVYTLPPSRDELTKNQVSLTCLV KGFYPSDIAVEWESNGQPENNYKTTPPVLDSDGSFFLYSKLTVDKSRWQQGNVFSC SVMHEALHNHYTQKSLSLSP (SEQ ID NO: 10) |

**Table 5**

| |
|---|
| • Hv1Lt1/Hv2Lt1 Light chain CDR1: |
| RASRAIRNNLH (SEQ ID NO: 11) |
| • Hv1Lt1/Hv2Lt1 Light chain CDR2: |
| LLIKYVSQSIS (SEQ ID NO: 12) |
| • Hv1Lt1/Hv2Lt1 Light chain CDR3: |
| QQSDSWPYT (SEQ ID NO: 13) |
| • Hv1Lt1/Hv2Lt1 Light chain signal peptide amino acid sequence: |
| MLPSQLIGFLLLWVPASRG (SEQ ID NO: 31) |
| • HvlLtl/Hv2Ltl Light chain variable region |
| EIVLTQSPDFQSVTPKEKVTITCRASRAIRNNLHWYQQKPDQSPKLLIKYVSQSISGV PSRFSGSGSGTDFTLTINSLEAEDAATYYCQQSDSWPYTFGQGTKLEIK (SEQ ID NO: 14) |
| • Hv1Lt1/Hv2Lt1 Light chain constant region |
|  (SEQ ID NO: 17) |
| • HvlLtl/Hv2Ltl Light chain: |
| MLPSQLIGFLLLWVPASRGEIVLTQSPDFQSVTPKEKVTITCRASRAIRNNLHWYQQ KPDQSPKLLIKYVSQSISGVPSRFSGSGSGTDFTLTINSLEAEDAATYYCQQSDSWPY TFGQGTKLEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSPVTKSFN RGEC (SEQ ID NO: 15) |

Tables 6 to 8 show the base sequence of the two obtained humanized anti-CKAP4 antibodies (Hv1Lt1 and Hv2Ltl). Table 6 shows the base sequence of a heavy chain of HvlLtl. Table 7 shows the base sequence of a heavy chain of Hv2Ltl. Table 8 shows the base sequence of light chains of Hv1Lt1 and Hv2Lt1 (the light chains of Hv1Lt1 and Hv2Lt1 have the same sequence).

**Table 6**

| |
|---|
| • Hv1Lt1 Heavy chain: |
| ATGGATTGGACCTGGCGGGTGTTCTGTCTGCTGGCTGTTGCTCCTGGCGCTCACTC TCAGGTTCAGCTGGTTCAGTCTGGCGCCGAAGTGAAGAAACCTGGCGCCTCTGTG AAGGTGTCCTGCAAGGCTTCCGGCTTCACCTTCACCAGCTACTGGATGCACTGGGT CCGACAGGCTCCAGGACAAGGCTTGGAGTGGATCGGCAACATCAACCCTTCTAAC GGCGGCACCAACTACAACGCCAAGTTCAAGTCCAGAGTGACCATGACCAGAGACA CCTCCACCAGCACCGTGTACATGGAACTGTCCAGCCTGAGATCCGAGGACACCGC CGTGTACTACTGTGCCAGATGGGTGTCCTTCAGCTATAGGGGCGCCATGGATTATT GGGGCCAGGGCACACTGGTCACCGTGTCATCTgccTCCACCAAGGGACCCTCTGTG TTTCCTCTGGCTCCCTCCAGCAAGTCTACCTCTGGTGGAACAGCTGCCCTGGGCTG CCTGGTCAAGGATTACTTTCCTGAGCCTGTGACCGTGTCCTGGAACTCTGGCGCTC TGACATCTGGCGTGCACACCTTTCCAGCTGTGCTGCAGTCCTCCGGCCTGTACTCT CTGTCCTCTGTCGTGACCGTGCCTTCCAGCTCTCTGGGCACCCAGACCTACATCTG CAATGTGAACCACAAGCCTTCCAACACCAAGGTGGACAAGAAGGTGgaaCCCAAGT CCTGCGACAAGACCCACACCTGTCCTCCATGTCCTgctCCAGAACTGCTCGGCGGAC CTTCCGTGTTCCTGTTTCCTCCAAAGCCTAAGGACACCCTGATGATCTCTCGGACC CCTGAAGTGACCTGCGTGGTGGTGGATGTGTCTCACGAGGATCCCGAAGTGAAGT TCAATTGGTACGTGGACGGCGTGGAAGTGCACAACGCCAAGACCAAGCCTAGAGA GGAACAGTACAACTCCACCTACAGAGTGGTGTCCGTGCTGACCGTGCTGCACCAG GATTGGCTGAACGGCAAAGAGTACAAGTGCAAGGTGTCCAACAAGGCCCTGCCTG CTCCTATCGAAAAGACCATCTCCAAGGCCAAGggcCAGCCTAGGGAACCCCAGGTTT ACACCTTGCCTCCATCTCGGGACGAGCTGACCAAGAACCAGGTGTCCCTGACCTG TCTCGTGAAGGGCTTCTACCCCTCCGATATCGCCGTGGAATGGGAGTCTAATGGCC AGCCTGAGAACAACTACAAGACAACCCCTCCTGTGCTGGACTCCGACGGCTCATTC TTCCTGTACTCCAAGCTGACAGTGGACAAGTCCAGATGGCAGCAGGGCAACGTGT TCTCCTGCTCCGTGATGCACGAGGCCCTGCACAATCACTACACCCAGAAGTCCCTG TCTCTGTCCCCTTGA (SEQ ID NO: 18) |

**Table 7**

| |
|---|
| • Hv2Lt1 Heavy chain: |
| ATGGATTGGACCTGGCGGGTGTTCTGTCTGCTGGCTGTTGCTCCTGGCGCTCACTC TCAGGTTCAGCTGGTTCAGTCTGGCGCCGAAGTGAAGAAACCTGGCGCCTCTGTG AAGGTGTCCTGCAAGGCTTCCGGCTACACCTTTACCAGCTACTGGATGCACTGGGT CCGACAGGCTCCAGGACAAGGCTTGGAGTGGATGGGCATCATCAACCCTTCTAAC GGCGGCACCAGCTACGCCCAGAAATTCCAGGGCAGAGTGACCATGACCAGAGACA CCTCCACCAGCACCGTGTACATGGAACTGTCCAGCCTGAGATCCGAGGACACCGC CGTGTACTACTGTGCCAGATGGGTGTCCTTCAGCTATAGGGGCGCCATGGATTATT GGGGCCAGGGCACACTGGTCACCGTGTCATCTgccTCCACCAAGGGACCCTCTGTG TTTCCTCTGGCTCCCTCCAGCAAGTCTACCTCTGGTGGAACAGCTGCCCTGGGCTG CCTGGTCAAGGATTACTTTCCTGAGCCTGTGACCGTGTCCTGGAACTCTGGCGCTC TGACATCTGGCGTGCACACCTTTCCAGCTGTGCTGCAGTCCTCCGGCCTGTACTCT CTGTCCTCTGTCGTGACCGTGCCTTCCAGCTCTCTGGGCACCCAGACCTACATCTG CAATGTGAACCACAAGCCTTCCAACACCAAGGTGGACAAGAAGGTGgaaCCCAAGT CCTGCGACAAGACCCACACCTGTCCTCCATGTCCTgctCCAGAACTGCTCGGCGGAC CTTCCGTGTTCCTGTTTCCTCCAAAGCCTAAGGACACCCTGATGATCTCTCGGACC CCTGAAGTGACCTGCGTGGTGGTGGATGTGTCTCACGAGGATCCCGAAGTGAAGT TCAATTGGTACGTGGACGGCGTGGAAGTGCACAACGCCAAGACCAAGCCTAGAGA GGAACAGTACAACTCCACCTACAGAGTGGTGTCCGTGCTGACCGTGCTGCACCAG GATTGGCTGAACGGCAAAGAGTACAAGTGCAAGGTGTCCAACAAGGCCCTGCCTG CTCCTATCGAAAAGACCATCTCCAAGGCCAAGggcCAGCCTAGGGAACCCCAGGTTT ACACCTTGCCTCCATCTCGGGACGAGCTGACCAAGAACCAGGTGTCCCTGACCTG TCTCGTGAAGGGCTTCTACCCCTCCGATATCGCCGTGGAATGGGAGTCTAATGGCC AGCCTGAGAACAACTACAAGACAACCCCTCCTGTGCTGGACTCCGACGGCTCATTC TTCCTGTACTCCAAGCTGACAGTGGACAAGTCCAGATGGCAGCAGGGCAACGTGT TCTCCTGCTCCGTGATGCACGAGGCCCTGCACAATCACTACACCCAGAAGTCCCTG TCTCTGTCCCCTTGA (SEQ ID NO: 19) |

**Table 8**

| |
|---|
| • Hv1Lt1/Hv2Lt1 Light chain: |
| ATGCTGCCCTCTCAGCTGATCGGCTTTCTGCTGCTGTGGGTGCCAGCTTCCAGAGG CGAGATTGTGCTGACCCAGTCTCCTGACTTCCAGAGCGTGACCCCTAAAGAGAAA GTCACCATCACCTGTCGGGCCTCTCGGGCCATCAGAAACAACCTGCACTGGTATCA GCAAAAACCTGATCAGTCCCCTAAGCTGCTGATCAAATACGTGTCCCAGTCCATCT CCGGCGTGCCCTCTAGATTTTCCGGCTCTGGCTCTGGCACCGACTTCACCCTGACC ATCAATTCCCTGGAAGCCGAGGATGCCGCCACCTACTACTGCCAGCAGTCTGACTC TTGGCCCTACACCTTTGGCCAGGGCACCAAGCTGGAAATCAAGcgaACAGTGGCTG CCCCTTCCGTGTTCATCTTCCCACCTTCCGACGAGCAGCTGAAGTCTGGCACAGCC TCTGTCGTGTGCCTGCTGAACAACTTCTACCCTCGGGAAGCCAAGGTGCAGTGGA AGGTGGACAATGCCCTGCAGTCCGGCAACTCCCAAGAGTCTGTGACCGAGCAGGA CTCCAAGGACAGCACCTACAGCCTGTCCTCCACACTGACCCTGTCCAAGGCCGACT ACGAGAAGCACAAGGTGTACGCCTGCGAAGTGACCCATCAGGGCCTGTCTAGCCC TGTGACCAAGTCTTTCAACCGGGGCGAGTGCTGA (SEQ ID NO: 20) |

### 2-4. In Vitro Characterization Analysis of Humanized Anti-CKAP4 Antibody

The affinity of humanized anti-CKAP4 antibodies to the extracellular region of CKAP4 was measured by Biacore assay. The measured association constants (kₐ) of the antibodies were 1.25 × 10⁵ for 3F11-2B10, 1.50 × 10⁴ for Hv1Lt1, and 3.79 × 10⁴ for Hv2Lt1 (Table 9). The k_{d} values were determined as 1.80 × 10⁻³ for 3F11-2B10, 1.14 × 10⁻⁵ for HvlLtl, and 1.70 × 10⁻⁶ for Hv2Ltl. The average KD values of these antibodies were 14.5 nM for 3F11-2B10, 0.76 nM for HvlLtl, and 0.05 nM for Hv2Lt1. These results suggest that the humanized anti-CKAP4 antibodies have a higher (by less than 10-fold) binding affinity to the extracellular domain of CKAP4 than the mouse anti-CKAP4 antibody.

**Table 9**

| **Antibody** | **ka (1/Ms)** | **kd (1/s)** | **KD (M)** |
|---|---|---|---|
| **Mouse antibody 3F11-2B10** | **1.25E+05** | **1.80E-03** | **1.45E-08** |
| **Humanized antibody Hv1Lt1** | **1.50E+04** | **1.14E-05** | **7.61E-10** |
| **Humanized antibody Hv2Ltl** | **3.79E+04** | **1.70E-06** | **4.49E-11** |

Further, two types of humanized anti-CKAP4 antibodies (hCKAP4 Ab) Hv1Lt1 and Hv2Lt1 detected CKAP4 in lysates of human S2-CP8 cells, canine kidney cells MDCK I, and mouse lung cancer cells Lewis Lung Carcinoma (LLC) with equivalent efficiency. Both types of humanized CKAP4 antibodies were able to immunoprecipitate endogenous CKAP4 from S2-CP8 cell lysates and efficiently detected CKAP4 present in the endoplasmic reticulum (ER) by immunocytochemistry. No background signal was detected in immunostaining of S2-CP8 cells in which CKAP4 was knocked out using the Crispr-Cas9 system (S2-CP8/CKAP4 KO). The results confirmed that the two types of humanized anti-CKAP4 antibodies specifically recognize CKAP4. Further, Hv1Lt1 detected CKAP4 on the cell surface. All humanized anti-CKAP4 antibodies inhibited the binding of DKK1 and CKAP4 in a dose-dependent manner in vitro. When S2-CP8 cells were treated with humanized anti-CKAP4 antibodies, AKT activity was inhibited by both antibodies.

### 2-5. Tumor Suppression Effect of Humanized Anti-CKAP4 Antibody In Vivo

To evaluate the in vivo tumor formation-suppressing effect of humanized anti-CKAP4 antibody Hv1Lt1, two types of human pancreatic cancer cell lines, S2-CP8 and MIA PaCa-2 cells, which express high levels of DKK1 and cell membrane CKAP4, were transplanted into immunodeficient mice, and Hv1Lt1 was injected intraperitoneally (Fig. 2A) or intravenously (Figs. 2B and 2C). The results show that in vivo xenograft tumor formation induced by both the cell lines was inhibited by the Hv1Lt1 injection. These results confirmed that the humanized anti-CKAP4 antibody Hv1Lt1 exhibited tumor formation-suppressing effects in vivo against various tumor cells, regardless of the administration route.

Next, to assess the non-specific toxicity of Hv1Lt1, human cervical cancer HeLa S3 cells, which expressed CKAP4 but did not express DKK1 on the cell surface, were treated with Hv1Lt1. Hv1Lt1 did not exhibit any inhibitory effect on the *in vitro* proliferation of HeLa S3 cells. In HeLa S3 cells treated with staurosporine, a known inducer of apoptosis, the nuclei were strongly stained with propidium iodide. In contrast, in HeLa S3 cells treated with Hv1Lt1, no nuclear staining with propidium iodide was observed, and apoptosis was not observed. Further, Hv1Lt1 or control IgG was intraperitoneally administered to wild-type immunodeficient C57BL/6 mice for 3 months to assess the long-term administration toxicity of the humanized anti-CKAP4 antibody. A therapeutic dose of Hv1Lt1 (280 µg/dose) was administered to two mice, and the same amount of IgG was injected into three mice. After 3 months of administration, no obvious changes were observed in macroscopic appearance (including coat condition and activity level) or body weight. Similarly, no abnormalities were observed in the macroscopic appearance or organ weight of major organs, such as the excised heart, lungs, liver, spleen, pancreas, stomach, small intestine, and kidneys. Sections of the excised organs were evaluated histologically, but no pathological abnormalities were observed. To examine the possibility that administered Hv1Lt1 deposits on endogenous CKAP4 in non-tumor tissue, tissue sections prepared from excised organs were stained with a secondary antibody that specifically recognizes human IgG. Since no deposition of human IgG was detected in the tissues, it was considered that Hv1Lt1 does not binding to endogenous CKAP4 in vivo. At the time of euthanasia, blood samples (both whole blood sample and serum) were collected and blood tests were performed. As a result of the complete blood count and clinical laboratory tests, no apparent abnormal values were observed compared to healthy mice and the control group (to which control IgG was administered), and the values were within the normal range (Table 10). The above results confirmed that the novel humanized CKAP4 antibody has no toxicity or side effects in vitro and in vivo.

**Table 10**

| | **Normal range** | **Healthy Control** | **IgG#1** | **IgG#2** | **IgG#3** | **Hv1Lt1#1** | **Hv1Lt1#2** |
|---|---|---|---|---|---|---|---|
| **RBC (10⁴/µL)** | **952±19.2** | **935** | **975** | **994** | **943** | **952** | **939** |
| **HGB (g/dL)** | **13.9±0.41** | **13.3** | **13.1** | **13.3** | **13.0** | **13.0** | **12.9** |
| **HCT (%)** | **47.3±1.14** | **42.4** | **44.0** | **45.2** | **43.8** | **42.4** | **42.9** |
| **CaPLT (10⁴/µL)** | **131.2±10.61** | **108.9** | **100.4** | **118.1** | **83.4** | **109.1** | **70.8** |
| **WBC (10²/µL)** | **25.6±8.89** | **10.7** | **25.1** | **9.6** | **20.8** | **12.8** | **17.9** |
| **LYMPH (%)** | | **80.4** | **80.9** | **76.0** | **67.8** | **72.7** | **62.6** |
| **NEUT (%)** | | **16.8** | **14.3** | **14.7** | **31.2** | **21.0** | **10.0** |
| **EO (%)** | | **1.9** | **4.0** | **8.3** | **0.5** | **5.5** | **24.0** |
| **BASO (%)** | | **0.0** | **0.0** | **3.0** | **0.0** | **0.0** | **0.0** |
| **MONO (%)** | | **0.9** | **0.8** | **1.0** | **0.5** | **0.8** | **3.4** |
| **TP (g/dL)** | **5.53±0.14** | **4.6** | **5.3** | **4.9** | **5.2** | **4.9** | **5.0** |
| **ALB (g/dL)** | **3.57±0.119** | **3.0** | **3.1** | **3.1** | **3.0** | **2.9** | **3.2** |
| **BUN (mg/dL)** | **26.3±3.09** | **23.8** | **27.7** | **25.4** | **23.7** | **26.0** | **24.4** |
| **CRE (mg/dL)** | **0.07±0.017** | **0.12** | **0.10** | **0.11** | **0.07** | **0.09** | **0.12** |
| **UA (mg/dL)** | | **0.6** | **0.7** | **0.6** | **0.3** | **1.2** | **1.0** |
| **Na (mEq/L)** | **149.3±1.26** | **146** | **149** | **150** | **151** | **150** | **142** |
| **K (mEq/L)** | **5.29±0.457** | **4.0** | **4.4** | **4.2** | **3.7** | **5.3** | **5.0** |
| Cl **(mEq/L)** | **109. 4 ±1.50** | **112** | **111** | **114** | **112** | **112** | **112** |
| **Ca (mg/dL)** | **9.0±0.18** | **9.0** | **19.4** | **9.5** | **9.5** | **9.3** | **8.6** |
| **IP (mg/dL)** | **7.7±0.60** | **5.8** | **6.3** | **8.2** | **10.6** | **7.3** | **7.4** |
| **AST (IU/L)** | **64±12.7** | **48** | **53** | **46** | **66** | **76** | **152** |
| **ALT(IU/L)** | **29±5.5** | **30** | **36** | **29** | **39** | **33** | **32** |
| **ALP (IU/L)** | **244±15.3** | **286** | **209** | **174** | **251** | **171** | **196** |
| **LDH (IU/L)** | | **188** | **135** | **120** | **118** | **245** | **478** |
| **AMY (IU/L)** | | **1978** | **2427** | **1879** | **1990** | **2340** | **2330** |
| **CK (IU/L)** | | **180** | **138** | **133** | **166** | **409** | **776** |
| **ChE (IU/L)** | | **24** | **28** | **24** | **27** | **28** | **26** |
| **T-CHO (mg/dL)** | **76±9.4** | **92** | **111** | **98** | **83** | **95** | **86** |
| **TG (mg/dL)** | **37±11.6** | **76** | **65** | **70** | **55** | **111** | **88** |
| **HDL-C (mg/dL)** | | **62** | **73** | **65** | **58** | **63** | **60** |
| T-BIL (mg/dL) | 0.17±0.034 | 0.04 | 0.05 | 0.05 | 0.08 | 0.04 | 0.04 |

### 2-6. In vitro and In Vivo Growth Inhibitory Effects of Hv1Lt1 on Pancreatic Cancer Organoids

In recent years, accumulating evidence has suggested that DKK1 may regulate antitumor immunity within the tumor microenvironment (TME). To elucidate the pharmacological activity of Hv1Lt1 in TME containing mesenchymal cell components, a mouse model was established. In this model, human pancreatic ductal-like adenocarcinoma (KPC tumors) that develops in genetically modified mice (LSL-KrasG12D/+; Trp53fl/fl; Elastase-Cre (KPC mice, in which pancreas-specific active mutant KRAS allele expression and Trp53 deficiency are induced)) is used. Organoids established from KPC tumors (KPC organoids) can be cultured continuously and can be transplanted into C57BL/6 immunodeficient mice either subcutaneously or orthotopically. In KPC/DKK1/CKAP4 organoids, DKK1 and CKAP4 were ectopically expressed (Fig. 4A). The DKK1-CKAP4 signal activated within the organoids promoted the organoid sphere proliferation (Fig. 4B). Further, in organoids that express CKAP4 but do not express DKK1, DKK1 promoted organoid sphere growth, whereas Hv1Lt1 inhibited this growth (Fig. 4C). When KPC/CKAP4/DKK1 organoids were surgically transplanted into the pancreatic tail of wild-type C57BL/6 mice to induce pancreatic tumors, the histological features of human pancreatic cancer were successfully reproduced (Fig. 4D). Hv1Lt1 suppressed KPC organoid-derived tumor growth by up to 60% (Fig. 4D).

### 2-7. Transcriptome Analysis of Pancreatic Tumors Treated with Hv1Lt1

Bulk RNA-sequencing analysis of the resected tumors revealed a clear separation between the Hv1Lt1-treated group and the control group, as demonstrated by hierarchical clustering and principal component analysis (Figs. 5A and 5B). Gene expression analysis identified 421 differentially expressed genes (DEGs) (including 88 upregulated and 333 downregulated genes in Hv1Lt1-treated tumors. (Fig. 5C). Enrichment analysis revealed that genes in signaling pathways relating to immune responses were significantly decreased in expression in the Hv1Lt1 treatment group (Fig. 5D). Furthermore, tissue sections were prepared from tumor samples (including samples subjected to bulk RNA-sequencing analysis) extracted from the control group and the Hv1Lt1 antibody-treated group, and immunohistochemical evaluation was performed. The results showed that in the Hv1Lt1 antibody-treated tumors, infiltration of CD8-positive T cells and dendritic (DC) cells, which are responsible for antitumor immune responses, was enhanced (Fig. 5E). These results, taken together, suggest that Hv1Lt1 administration not only suppresses autonomous proliferation of tumor cells, but also participates in the regulation of antitumor immunity via the TME in pancreatic cancer.

### 2-8. Tumor suppression Effect In Vitro and In Vivo by Combination of Humanized Anti-CKAP4 Antibody with Other Therapeutic Agents

In recent years, various molecular targeted therapies, including antibody-based drugs, have been developed. Among these newly developed drugs, some exert synergistic therapeutic effects when combined with existing drugs, thereby improving the prognosis of cancer patients. Accordingly, in an in vitro sphere-formation assay using S2-CP8 cells, Hv1Lt1 and either gemcitabine (Fig. 6A) or nanoparticle albumin-bound paclitaxel (nab-PTX) (Fig. 6B) were added to the liquid culture medium, individually or in combination. The combined treatment produced an additive inhibitory effect on sphere formation. Further, Hv1Lt1 and either gemcitabine (Figs. 3A and 6C) or nanoparticle albumin-based paclitaxel (nab-PTX) (Figs. 3B and 6D) were administered in combination to model mice with xenograft tumors derived from S2-CP8 cells. All of these drugs have been used as standard therapeutic agents for human pancreatic cancer. Hv1Lt1, gemcitabine, and nab-PTX each demonstrated antitumor effects in vivo as single agents, and their combination therapy showed stronger tumor growth inhibitory effects against S2-CP8 cell-induced xenograft tumor formation than each single agent therapy (Figs. 3A, 3B, 6C, and 6D). Furthermore, combination therapy of Hv1Lt1 and lenvatinib against Hep3B cell-induced xenograft tumor formation also showed significant synergistic antitumor effects (Fig. 3C). These results strongly support the potential of humanized anti-CKAP4 antibody combination therapy for human pancreatic cancer and liver cancer in future clinical applications.

## Claims

1. A humanized anti-CKAP4 antibody, or an antigen-binding fragment thereof, comprising:
a heavy chain variable region that comprises
a heavy chain CDR1 comprising the amino acid sequence represented by SEQ ID NO: 1 or 6,
a heavy chain CDR2 comprising the amino acid sequence represented by SEQ ID NO: 2 or 7, and
a heavy chain CDR3 comprising the amino acid sequence represented by SEQ ID NO: 3 or 8, and that comprises
an amino acid sequence having at least 90% identity to the amino acid sequence represented by SEQ ID NO: 4 or 9; and
a light chain variable region that comprises
a light chain CDR1 comprising the amino acid sequence represented by SEQ ID NO: 11,
a light chain CDR2 comprising the amino acid sequence represented by SEQ ID NO: 12, and
a light chain CDR3 comprising the amino acid sequence represented by SEQ ID NO: 13, and that comprises
an amino acid sequence having at least 90% identity to the amino acid sequence represented by SEQ ID NO: 14.

2. The humanized anti-CKAP4 antibody or antigen-binding fragment thereof according to claim 1, wherein at least one of the identities is at least 95%.

3. The humanized anti-CKAP4 antibody or antigen-binding fragment thereof according to claim 1, wherein at least one of the identities is 100%.

4. The humanized anti-CKAP4 antibody or antigen-binding fragment thereof according to claim 1, comprising
a heavy chain variable region that comprises the amino acid sequence represented by SEQ ID NO: 4 or 9, and
a light chain variable region that comprises the amino acid sequence represented by SEQ ID NO: 14.

5. The humanized anti-CKAP4 antibody or antigen-binding fragment thereof according to claim 1,
wherein
the heavy chain variable region comprises the amino acid sequence represented by SEQ ID NO: 4 or 9, and
the light chain variable region comprises the amino acid sequence represented by SEQ ID NO: 14.

6. The humanized anti-CKAP4 antibody or antigen-binding fragment thereof according to any one of claims 1 to 4, comprising
a heavy chain constant region that comprises an amino acid sequence having at least 90% identity to the amino acid sequence represented by SEQ ID NO: 16, and
a light chain constant region that comprises an amino acid sequence having at least 90% identity to the amino acid sequence represented by SEQ ID NO: 17.
6 A polynucleotide comprising a coding sequence for the humanized anti-CKAP4 antibody or antigen-binding fragment thereof of any one of claims 1 to 4.

7. The polynucleotide according to claim 6, which is an expression vector.

8. A cell comprising the polynucleotide of claim 6.

9. A pharmaceutical composition comprising the humanized anti-CKAP4 antibody or antigen-binding fragment thereof of any one of claims 1 to 4.

10. A reagent comprising the humanized anti-CKAP4 antibody or antigen-binding fragment thereof of any one of claims 1 to 4.

11. An antitumor agent comprising the humanized anti-CKAP4 antibody or antigen-binding fragment thereof of any one of claims 1 to 4.

12. The antitumor agent according to claim 11, wherein the humanized anti-CKAP4 antibody or antigen-binding fragment thereof is a conjugate with a drug, or is for use in co-administration with a drug.

13. An antitumor immune response activator comprising the humanized anti-CKAP4 antibody or antigen-binding fragment thereof of any one of claims 1 to 4.
